# EUROPEAN PATENT SPECIFICATION

(11) **EP 1 753 407 B1**
(45) Date of publication and mention of the grant of the patent: **08.03.2017**
(21) Application number: 05760431.6
(22) Date of filing: 07.06.2005
(51) Int. Cl.: A61K 9/20, A61K 31/565

(54) **SUGAR COATINGS AND METHODS THEREFOR**
ZUCKERÜBERZÜGE UND VERFAHREN DAFÜR
ENROBAGES DRAGÉIFIÉS ET PROCÉDÉS PERMETTANT DE PRODUIRE CES ENROBAGES DRAGÉIFIÉS

(30) Priority: 07.06.2004 US 577668 P
(43) Date of publication of application: 21.02.2007
(73) Proprietor: Wyeth LLC, New York, NY 10017-5755 (US)
(72) Inventor: CLARK, John, C., Peru, NY 12972 (US); MICHELUCCI, John, J., Plattsburgh, NY 12901 (US); SHERMAN, Deborah, M., Plattsburgh, NY 12901 (US)
(74) Representative: Pfizer
(86) International application number: PCT/US2005/019972
(87) International publication number: WO 2005/120466

(56) References cited:
- EP-A1- 0 803 250
- WO-A-95/06462
- WO-A-97/04753
- WO-A-99/03449
- US-A- 3 487 152
- US-A1- 2003 203 098
- US-B1- 6 630 166
- MOLDENHAUER ET AL.: "Untersuchungen über den Einsatz von Polyvinylalkohol (PVA) zur Erzielung zuckerhaltiger und zuckerfreier Drageeüberzüge" PHARMAZIE, vol. 29, no. 1, 1974, pages 26-30, XP008069938

## Description

### FIELD OF THE INVENTION

The invention is directed generally to the field of pharmaceutical formulations. More specifically, the invention relates to sugar-containing compositions suitable for use in coating solid preparations such as tablets, pills, granules and grains. Methods of using such coatings are provided, as are solid dosage forms coated with the compositions.

### BACKGROUND OF THE INVENTION

Solid pharmaceutical dosage forms, most notably tablets, have been coated using a wide variety of materials and processes. The reasons for this include the aesthetic as well as the practical. For example, tablet coatings can mask an unpleasant taste or odor, can increase ease of ingestion by the patient and can serve to improve the ultimate appearance of the dosage form. Similarly, coatings can protect a product from the effects of air, moisture and light, can improve product identification and can facilitate handling in packaging and filling lines during manufacture.

Typically, pharmaceutical dosage forms can be sugar coated or film coated. Conventional sugar coating compositions have been prepared as aqueous solutions of sugar (syrups), most commonly sucrose, and it is said that tablets with excellent hermetic properties and smooth surface are obtained by the formation of tight, block-form structures of sugar crystals. Additional agents that may be included in sugar-coated tablets include talc (used as a sugar coating dispersion agent), precipitated calcium carbonate (used as a sugar coating suspension agent), gelatin, gum arabic and pullulan (used as sugar coating binders), and carnauba wax (often applied as a sugar coating glossing agent).

Unfortunately, sugar-coating is a multi-step and tedious process, and is highly dependent on the use of skilled manpower. The typical sugar-coating process can be subdivided into six main steps: (1) sealing; (2) subcoating; (3) smoothing; (4) color coating; (5) polishing; and (6) printing. In the hands of a skilled worker, sugar coated products are elegant in appearance, but certain problems beset the process and the ultimate product. For example, the sugar coating process requires that the tablets be kept constantly tumbling, thus presenting difficulties such as fragmenting of those units not strong enough to withstand the stress encountered. Also, color nonuniformity, rough or overly soft coatings and/or marbling may present additional problems to be addressed.

In an attempt to overcome some of these difficulties, film coating was introduced to the pharmaceutical industry in the 1950's. Film coating involves the deposition of a thin, uniform, typically polymeric membrane to the substrate, usually by a spray technique. Advantages of the film coating process include minimal weight increase of the ultimate dosage form, reduction in processing times, and improved resistance to chipping.

Film coatings have also been utilized to modify the release of the constituents of the core tablet as, for example, via enteric coatings in which the polymer employed in the coating is essentially impervious to gastric pH but is soluble in the increased pH of the intestines. A further application of film coating lies in the formulation of extended-release coatings which help eliminate the need for multiple dose regimens of a particular therapeutic agent. Various types of extended release approaches are known. One such approach is a diffusion coating, which involves depositing a coating (usually from an organic solvent) on a soluble substrate core with a porous membrane that is water-permeable but water-insoluble. The release profile of the therapeutic agent can be modified by the inclusion of water-soluble substances within the membrane. These substances are dissolved by the gastrointestinal fluids, thereby creating pores within the film. These pores allow the gastrointestinal fluids to pass through the membrane and dissolve the therapeutic agent within the tablet core. The diffusion rate can be controlled by the thickness arid composition of the diffusion membrane. For this system to function properly, the constituents of the diffusion coating formulation should exhibit good mechanical strength and flexibility. Unfortunately, however, such coating formulations, when deposited on the substrate, frequently lack the requisite mechanical strength and flexibility, thereby leading to rupture of the deposited film during dissolution in the gastrointestinal tract. This, in turn, permits the sudden release of the entire contents of the substrate containing the therapeutic agent in a phenomenon referred to as "dose dumping". Such a situation is clearly undesirable for extended-release dosage forms given the higher amount of therapeutic agent found therein as compared to conventional formulations.

Additionally, notwithstanding the advantages that film-coating provides, certain difficulties attend the film coating process, including the tendency to laminate if the tablets being coated are not of sufficient strength, the inability to hide defects in the tablet core, mottling and the like. Ironically, the use of organic solvents in film coating, which permits a number of process advantages, also presents some of the major disadvantages. Due to their volatility, the use of organic solvents in the film coating process can lead to flammability hazards as well as concerns over environmental effects and potential toxicity to the operators. Organic solvents also add to the cost of the overall process, due to the costs of the solvents *per se* or costs encountered in reducing any potential hazards thereof.

Moreover, film-coating may not be suitable for tablets that contain a particularly hygroscopic core that is apt to swell either during processing or storage. For example, film-coated tablets with hydrogel cores, which contain relatively high percentages of water-soluble cellulosic materials in the tablet cores, have a tendency to crack. Tablets with cracked coatings are unacceptable, from both an aesthetic and functional standpoint; the elegant appearance, ease of ingestion, and odor-masking properties are diminished, and the active ingredient in the tablet cores may become exposed to environmental conditions detrimental to product stability.

The present invention addresses one or more of these problems by providing coatings that exhibit good mechanical strength and flexibility when applied to a substrate, thereby reducing the possibility of cracking. The coating formulation of the present invention also utilizes an aqueous solvent system thereby eliminating or at least minimizing the use of organic solvents in the film coating process delineated above. At the same time, the compositions generally can be applied using traditional film-coating equipment, and thus may be utilized in simpler and less labor-intensive methods than are necessary to produce the sugar-coated tablets of the prior art.

### SUMMARY OF THE INVENTION

In one embodiment, the invention is directed to compositions comprising water and a solids component that comprises: from 30 weight % to 60 weight % of at least one sugar; from 5 weight % to 10 weight % of at least one binder; from 3 weight % to 10 weight % of at least one hydroxyalkyl cellulose; from 15 weight % to 50 weight % of at least one water soluble polymer; the therapeutic agent medroxyprogesterone acetate in an amount of up to 3 weight%, a second polymer that is water soluble or water dispersible, in an amount of up to 20 weight %; and optionally, at least one plasticizer in an amount of up to 8 weight %; wherein the sugar comprises sucrose; the binder comprises microcrystalline cellulose, the hydroxyalkyl cellulose comprises hydroxypropyl cellulose; the water-soluble polymer comprises hydroxypropyl methyl cellulose; the second polymer comprises a polymethacrylate; and the plasticizer, when present, comprises polyethylene glycol.

The invention is also directed to solid dosage forms comprising a core material and one or more coatings disposed thereon, wherein the coating comprises: from 30 weight % to 60 weight % of at least one sugar; from 5 weight % to 10 weight % of at least one binder; from 3 weight % to 10 weight % of at least one hydroxyalkyl cellulose; from 15 weight % to 50 weight % of at least one water soluble polymer; the therapeutic agent medroxyprogesterone acetate in an amount of up to 3 weight %; a second polymer that is water soluble or water dispersible, in an amount of up to 20 weight %; and optionally, at least one plasticizer in an amount of up to about 8 weight % ; wherein, in the coating, the sugar comprises sucrose; the binder comprises microcrystalline cellulose, the hydroxyalkyl cellulose comprises hydroxypropyl cellulose; the water-soluble polymer comprises hydroxypropyl methyl cellulose; the second polymer comprises a polymethacrylate; and the plasticizer comprises polyethylene glycol.

In another embodiment, the invention is directed to a process comprising: providing a tablet core; spraying or otherwise applying to said tablet core a sugar coating composition comprising water and a solids component that comprises: from 30 weight % to 60 weight % of at least one sugar; from 5 weight % to 10 weight % of at least one binder; from 3 weight % to 10 weight % of at least one hydroxyalkyl cellulose; from 15 weight % to 50 weight % of at least one water soluble polymer; the therapeutic agent medroxyprogesterone acetate in an amount of up to 3 weight %; a second polymer that is water soluble or water dispersible, in an amount of up to 20 weight %; and optionally, at least one plasticizer in an amount of up to about 8 weight % ; wherein, the sugar comprises sucrose; the binder comprises microcrystalline cellulose, the hydroxyalkyl cellulose comprises hydroxypropyl cellulose; the water-soluble polymer comprises hydroxypropyl methyl cellulose; the second polymer comprises a polymethacrylate; and the plasticizer, when present, comprises polyethylene glycol; resulting in a coated tablet core.

### DESCRIPTION OF THE DRAWINGS

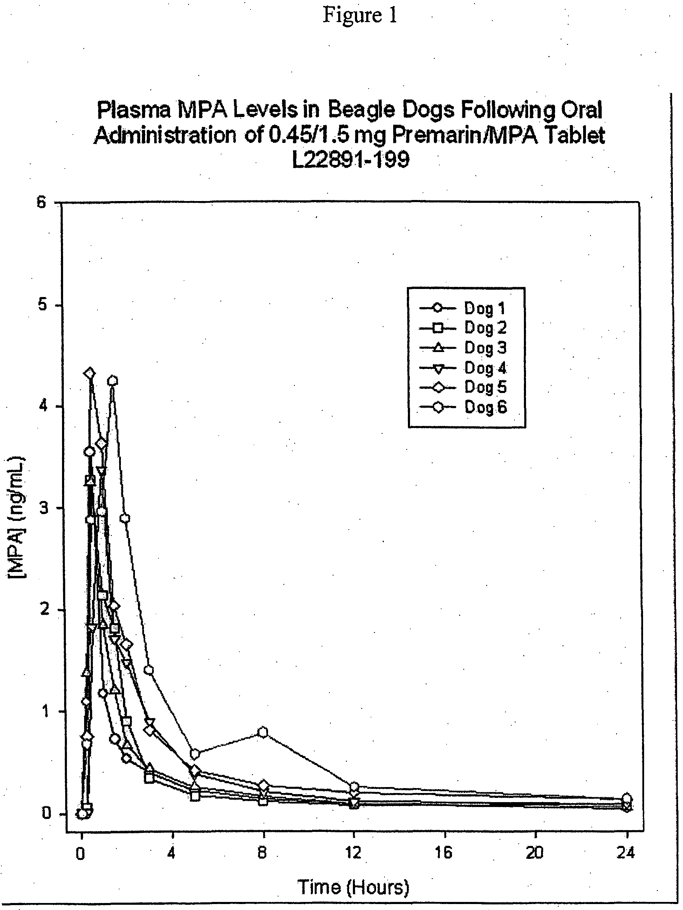

**Figure 1** shows plasma MPA levels in six beagle dogs following oral administration of the tablet formulation described in Example 3.

### DETAILED DESCRIPTION OF THE INVENTION

Concentrations, amounts, percentages, and other numerical data may be expressed or presented herein in a range format. It is to be understood that such a range format is used merely for convenience and brevity and thus, should be interpreted flexibly to include not only the numerical values explicitly recited as the limits of the range, but also, to include each of the individual numerical values or sub-ranges encompassed within that range as if each numerical value and sub-range is explicitly recited.

As an illustration, a concentration range of " about 1 weight % to about 10 weight %" should be interpreted to include not only the explicitly recited concentration of about 1 weight % to about 10 weight %, but also individual concentrations and the sub-ranges within the indicated range. Thus, included in this numerical range are individual concentrations such as 2 weight %, 5 weight %, and 8 weight %, and sub-ranges such as from 1 weight % to 3 weight %, from 5 weight % to 9 weight %, etc. The same principle applies to ranges reciting only one numerical value.

Similarly, an open ended range recited as "less than about 10 weight %" should be interpreted to include all of the values and ranges as elaborated above. Furthermore, it is understood that functional limitations may exist for limits not expressly recited by an open ended range, and that such limitations are included inherently as part of the disclosure of the present application, though not expressly recited. Such an interpretation should apply regardless of the breadth of the range or the characteristics being described.

In one embodiment, the invention is directed to compositions suitable for use in coating a solid preparation, such as a tablet, pill, granule, or the like. The compositions comprise water, and a solids component, and also include a therapeutic agent. Up to about 50% by weight of water may be replaced by another solvent, such as an alcohol. Preferably, the composition is in the form of an aqueous suspension, obtained by combining from about 30 weight % to about 98 weight % water, and from about 2 weight % to about 70 weight % of the solids component. In certain embodiments, the composition comprises from about 75 weight % to about 85 weight % water and from about 15 weight % to about 25 weight % of the solids component. In one such embodiment, the composition comprises about 82 weight % water.

The solids component contains, *inter alia,* one or more sugars. As used herein, the term "sugar" refers to any type of simple carbohydrate, such as a mono or disaccharide, either naturally obtained, refined from a natural source, or artificially produced, and includes, without limitation, sucrose, dextrose, maltose, glucose, fructose, galactose, mannose, lactose, trehalose, lactulose, levulose, raffinose, ribose, and xylose. The term "sugar," as used herein, also includes various "sugar substitutes" widely known to those of ordinary skill in the art of preparing solid dosage forms, such as the polyhydric alcohols (sometimes referred to as "sugar alcohols" or hydrogenated saccharides), for example sorbitol, mannitol, xylitol, and erythritol, and the sugar derivatives of polyhydric alcohols, such as maltitol, lactitol, isomalt, and polyalditol. Accordingly, the recitation of the term "sugar" generically should be interpreted to include such specific compounds, as well as others not expressly recited.

Unlike traditional sugar coatings, which are produced using syrups that contain upwards of 80 weight % sugars, on a dry weight basis, the compositions of the present invention contain a solids component that comprises from 30 weight % to 60 weight % of at least one sugar, and comprises sucrose. In some embodiments, the solids component contains from 35 weight % to 55 weight % sugar. In still other embodiments, the solids component contains from 35 to 45 weight % sugar.

The solids component also contains a binder, comprising microcrystalline cellulose, in an amount of from 5 weight % to 10 weight %. In some embodiments, the binder is present in an amount of from 5.5 weight % to 9 weight %, or in further embodiments, in an amount of from 5.5 to 7.5 weight %.

The solids component further contains from 3 weight % to 10 weight % of at least one hydroxyalkyl cellulose in which the alkyl group has from between one and ten carbon atoms. Representative hydroxyalkyl celluloses, without limitation, include hydroxyethyl cellulose (HEC) and hydroxypropyl cellulose (HPC),. The hydroxyalkyl cellulose comprises HPC. In some embodiments, the
solids component comprises from 4 weight % to 7 weight % hydroxyalkyl celluloses; and in further embodiments, from 4 to 5.5 weight % hydroxyalkyl celluloses.

In addition, the solids component contains from 15 weight % to 50 weight % of at least one water soluble polymer. Water soluble polymers suitable for use in coatings and as excipients in pharmaceutical dosage forms are widely known to those of skill in the art, and include, *inter alia,* Hypromellose (hydroxypropyl methylcellulose), acacia, sodium carboxymethylcellulose, dextrin, alginic acid, gelatin, guar gum, methylcellulose, sodium alginate, zein, polyvinylpyrrolidone, vinylpyrrolidine-vinyl acetate copolymer, vinyl acetate-crotonic acid copolymer and ethyl acrylate-methacrylate acid copolymer. The water-soluble polymer comprises Hypromellose. The specific amount of water soluble polymer to be used in the solids component varies, depending upon the polymer selected. In some embodiments, the water-soluble polymer is present in an amount from 25 weight % to 40 weight %, and further, in an amount from 20 weight % to 30 weight %.

The solids component of the compositions optionally contains at least one plasticizer. Suitable plasticizers are also well known to those skilled in the art, and include, for example, propylene glycol, glycerin, trimethylolpropane, polyethylene glycol (PEG) polymers, dibutyl sebacate, acetylated monoglycerides, diethylphthalate, triacetin, glyceryltriacetate, acetyltrietyhyl citrate and triethyl citrate. In certain embodiments, a PEG polymer is used. Such polymers are available commercially by grades of average molecular weight such PEG 100 to PEG 4,000. In some embodiments, PEG 400 is used. The plasticizer is present in the solid components in an amount of from 0 weight % to 8 weight %. In some embodiments, the plasticizer is present in an amount from 5 weight % to 7 weight %, or from 2 weight % to 4 weight % or from 2.5 to 3.5 weight %.

The solids component of the compositions also contains a second polymer, which is water soluble, or which is water dispersible, i.e. capable of forming an aqueous dispersion.
The second polymer comprises a polymethacrylate such as Eudragit^{®} NE30D, NE40D, RS30D and RL30D (Degussa Corp., Parsippany, NJ) and Kollicoat^{®} MAE30DP (BASF Corp.). In some embodiments, the second polymer may be a mixture of one or more suitable polymers, for example a mixture of RS30D and RL30D; and thus, the solids component of the compositions would comprise at least two additional polymers. In some embodiments, the polymethacrylate contains neutral methyacrylic acid esters. In some embodiments, the polymethacrylate contains neutral methyacrylic acid esters having trimethylammonioethyl methacrylate chloride in a molar ratio of quaternary ammonium groups to neutral ester groups of 1:20; or in further embodiments, of 1:40. In some embodiments, the polymethacrylate contains neutral methyacrylic acid esters without any functional groups. As used herein, the term "functional groups" refers to atoms or small groups of atoms (for example, two to four atoms) that exhibit a characteristic reactivity when treated with certain reagents; for example, and without limitation, the term includes, alkanes, alkenes, alkynes, alkyl halides, benzene, amines, ammonium, ethers, alcohols, aldehydes, ketones and carboxylic acids. In some embodiments, the second polymer that is water soluble or water dispersible is in an amount from 3 weight % to 20 weight % based on the percentage of solids in the solids component of the composition.

The compositions may also include optional ingredients, such as other flavoring agents, artificial sweeteners, color pigments, lubricants, glidants, surfactants. The selection of such alternative ingredients is within the skill of those in the art.

As noted previously, the compositions comprise medroxyprogesterone acetate and may also include one or more other therapeutic agents. As used herein, the term "therapeutic agent" refers also to a substance which is capable of exerting a therapeutic biological effect *in vivo.* The therapeutic agents may be neutral or positively or negatively charged. Examples of suitable pharmaceutical agents include, *inter alia,* diagnostic agents, pharmaceuticals, drugs, synthetic organic molecules, proteins, peptides, vitamins, and steroids. For example, the composition may include one or more hormonal steroids, such as medroxyprogesterone acetate, levonorgestrel, gestodene, medrogestone, estradiol, estriol, ethinylestradiol, mestranol, estrone, dienestrol, hexestrol, diethylstilbestrol, progesterone, desogestrel, norgestimate, hydroxyprogesterone, norethindrone, norethindone acetate, norgestrel, megestrol acetate, methyltestosterone, ethylestrenol, methandienone, oxandrolone, trimegestone, dionogest, and the like. Additionally, tissue selective progesterones and/or progesterone antagonists which may or may not have the typical steroidal functionality may be present in the composition. These include, but are not limited to: RU-486 (mifepristone), ZK 98 299 (onapristone), ZK-137316 (Schering AG, Berlin), ZK-230211 (Schering AG, Berlin), and HRP-2000 (17-acetoxy-[11ß-(4-N,N-dimethylaminophenyl)]-19-norpregna-4,9-diene-3,20-dione). Where desired, estrogenic steroids and progestogenic steroids may be used in combination.

The composition of the present invention is particularly suitable for use in coating a core material, to produce a solid dosage form. The term "core material" refers to any tablet, caplet, particle, micronized particle, particulate, pellet, pill, core, powder, granule, granulate, small mass, seed, specks, spheres, crystals, beads, agglomerates, mixtures thereof and the like. Typically, the preferred core material will be in a form sufficiently stable physically and chemically to be effectively coated in a system that involves some movement of the tablet, as for example in a perforated coating pan.

In a preferred embodiment, the core material is present in the form of a tablet. As used herein, the term "tablet" refers to a solid pharmaceutical dosage form containing a therapeutic agent with or without suitable diluents and prepared by either compression or molding methods, as are well known to those of ordinary skill in the art. Suitable methods of forming tablets are described, for example, in Edward M Rudnick, et al., Oral Solid Dosage Forms, in Remington: The Science and Practice of Pharmacy, 20th Ed., Chap. 45, Alfonso R. Gennaro, ed., Philadelphia College of Pharmacy and Science, Philadelphia, PA (2000). In more preferred embodiments, the core material is a tablet formed by compression methods.

Most frequently, the core material will comprise at least one therapeutic agent, as defined previously, and at least one pharmaceutically acceptable excipient. The term "pharmaceutically acceptable," as used herein, refers to materials that are generally not toxic or injurious to a patient when used in the compositions of the present invention, including when the compositions are administered by the oral route. The term "patient," as used herein, refers to animals, including mammals, preferably humans. "Excipients," as that term is used herein, refers to ingredients that provide bulk, impart satisfactory processing and compression characteristics, help control the dissolution rate, and/or otherwise give additional desirable physical characteristics to the core material. Included within this term, for example, are diluents, binders, lubricants and disintegrants well known to those of ordinary skill in the art, as described, for example, in the Handbook of Pharmaceutical Excipients, American Pharmaceutical Association, Washington, D.C. and The Pharmaceutical Society of Great Britain, London, England, (1986). Suitable excipients may include, for example, cellulosic material, such as, Hypromellose, HPC, HEC, carboxymethylcellulose, microcrystalline cellulose, ethyl cellulose, methyl cellulose, and their derivatives and salts; other organic compounds, such as PEG, talc, lactose and other sugars (as described above), acacia, dextrin, alginic acid, ethylcellulose resin, gelatin, guar gum, methylcellulose, pregelatinized starch, sodium alginate, starch, zein, polyvinylpyrrolidone, vinylpyrrolidine-vinyl acetate copolymer, vinyl acetate-crotonic acid copolymer and ethyl acrylate-methacrylate acid copolymer; plasticizers, such as propylene glycol, glycerin, trimethylolpropane, PEG polymers, dibutyl sebacate, acetylated monoglycerides, diethylphthalate, triacetin, glyceryltriacetate, acetyltrietyhyl citrate and triethyl citrate; and lubricants, such as talc, magnesium stearate, calcium stearate, stearic acid, hydrogenated vegetable oils, magnesium lauryl sulfate, sodium benzoate, a mixture of sodium benzoate and sodium acetate, sodium chloride, leucine, and Carbowax® 4000.

A wide variety of therapeutic agents may be utilized in the core material. Specific examples of therapeutic agents include, but are not limited to: acetazolamide, acetohexamide, acrivastine, alatrofloxacin, albuterol, alclofenac, aloxiprin, alprostadil, amodiaquine, amphotericin, amylobarbital, aspirin, atorvastatin, atovaquone, baclofen, barbital, benazepril, bezafibrate, bromfenac, bumetanide, butobarbital, candesartan, capsaicin, captopril, cefazolin, celecoxib, cephadrine, cephalexin, cerivastatin, cetrizine, chlorambucil, chlorothiazide, chlorpropamide, chlorthalidone, cinoxacin, ciprofloxacin, clinofibrate, cloxacillin, cromoglicate, cromolyn, dantrolene, dichlorophen, diclofenac, dicloxacillin, dicumarol, diflunisal, dimenhydrinate, divalproex, docusate, dronabinol, enoximone, enalapril, enoxacin, enrofloxacin, epalrestat, eposartan, essential fatty acids, estramustine, ethacrynic acid, ethotoin, etodolac, etoposide, fenbufen, fenoprofen, fexofenadine, fluconazole, flurbiprofen, fluvastatin, fosinopril, fosphenytoin, fumagillin, furosemide, gabapentin, gemfibrozil, gliclazide, glipizide, glybenclamide, glyburide, glimepiride, grepafloxacin, ibufenac, ibuprofen, imipenem, indomethacin, irbesartan, isotretinoin, ketoprofen, ketorolac, lamotrigine, levofloxacin, lisinopril, lomefloxacin, losartan, lovastatin, meclofenamic acid, mefenamic acid, mesalamine, methotrexate, metolazone, montelukast, nalidixic acid, naproxen, natamycin, nimesulide, nitrofurantoin, non-essential fatty acids, norfloxacin, nystatin, ofloxacin, oxacillin, oxaprozin, oxyphenbutazone, penicillins, pentobarbital, perfloxacin, phenobarbital, phenytoin, pioglitazone, piroxicam, pramipexol, pranlukast, pravastatin, probenecid, probucol, propofol, propylthiouracil, quinapril, rabeprazole, repaglinide, rifampin, rifapentine, sparfloxacin, sulfabenzamide, sulfacetamide, sulfadiazine, sulfadoxine, sulfamerazine, sulfamethoxazole, sulfafurazole, sulfapyridine, sulfasalazine, sulindac, sulphasalazine, sulthiame, telmisartan, teniposide, terbutaline, tetrahydrocannabinol, tirofiban, tolazamide, tolbutamide, tolcapone, tolmetin, tretinoin, troglitazone, trovafloxacin, undecenoic acid, ursodeoxycholic acid, valproic acid, valsartan, vancomycin, verteporfin, vigabatrin, vitamin K-S (II) and zafirlukast. Additional therapeutic agents include abacavir, acebutolol, acrivastine, alatrofloxacin, albuterol, albendazole, alfentanil, alprazolam, alprenolol, amantadine, amiloride, aminoglutethimide, amiodarone, amitriptyline, amlodipine, amodiaquine, amoxapine, amphetamine, amphotericin, amprenavir, amrinone, amsacrine, apomorphine, astemizole, atenolol, atropine, azathioprine, azelastine, azithromycin, baclofen, benethamine, benidipine, benzhexol, benznidazole, benztropine, biperiden, bisacodyl, bisanthrene, bromazepam, bromocriptine, bromperidol, brompheniramine, brotizolam, bupropion, butenafine, butoconazole, cambendazole, camptothecin, carbinoxamine, cephadrine, cephalexin, cetrizine, cinnarizine, chlorambucil, chlorpheniramine, chlorproguanil, chlordiazepoxide, chlorpromazine, chlorprothixene, chloroquine, cimetidine, ciprofloxacin, cisapride, citalopram, clarithromycin, clemastine, clemizole, clenbuterol, clofazimine, clomiphene, clonazepam, clopidogrel, clozapine, clotiazepam, clotrimazole, codeine, cyclizine, cyproheptadine, dacarbazine, darodipine, decoquinate, delavirdine, demeclo-cycline, dexamphetamine, dexchlorpheniramine, dexfenfluramine, diamorphine, diazepam, diethylpropion, dihydrocodeine, dihydroergotamine, diltiazem, dimenhydrinate, diphenhydramine, diphenoxylate, diphenyl-imidazole, diphenylpyraline, dipyridamole, dirithromycin, disopyramide, dolasetron, domperidone, donepezil, doxazosin, doxycycline, droperidol, econazole, efavirenz, ellipticine, enalapril, enoxacin, enrofloxacin, eperisone, ephedrine, ergotamine, erythromycin, ethambutol, ethionamide, ethopropazine, etoperidone, famotidine, felodipine, fenbendazole, fenfluramine, fenoldopam, fentanyl, fexofenadine, flecainide, flucytosine, flunarizine, flunitrazepam, fluopromazine, fluoxetine, fluphenthixol, fluphenthixol decanoate, fluphenazine, fluphenazine decanoate, flurazepam, flurithromycin, frovatriptan, gabapentin, granisetron, grepafloxacin, guanabenz, halofantrine, haloperidol, hyoscyamine, imipenem, indinavir, irinotecan, isoxazole, isradipine, itraconazole, ketoconazole, ketotifen, labetalol, lamivudine, lanosprazole, leflunomide, levofloxacin, lisinopril, lomefloxacin, loperamide, loratadine, lorazepam, lormetazepam, lysuride, mepacrine, maprotiline, mazindol, mebendazole, meclizine, medazepam, mefloquine, melonicam, meptazinol, mercaptopurine, mesalamine, mesoridazine, metformin, methadone, methaqualone, methylphenidate, methylphenobarbital, methysergide, metoclopramide, metoprolol, metronidazole, mianserin, miconazole, midazolam, miglitol, minoxidil, mitomycins, mitoxantrone, modafinil, molindone, montelukast, morphine, moxifloxacin, nadolol, nalbuphine, naratriptan, natamycin, nefazodone, nelfinavir, nevirapine, nicardipine, nicotine, nifedipine, nimodipine, nimorazole, nisoldipine, nitrazepam, nitrofurazone, nizatidine, norfloxacin, nortriptyline, nystatin, ofloxacin, olanzapine, omeprazole, ondansetron, omidazole, oxamniquine, oxantel, oxatomide, oxazepam, oxfendazole, oxiconazole, oxprenolol, oxybutynin, oxyphencyclimine, paroxetine, pentazocine, pentoxifylline, perchlorperazine, perfloxacin, perphenazine, phenbenzamine, pheniramine, phenoxybenzamine, phentermine, physostigmine, pimozide, pindolol, pizotifen, pramipexol, pranlukast, praziquantel, prazosin, procarbazine, prochlorperazine, proguanil, propranolol, pseudoephedrine, pyrantel, pyrimethamine, quetiapine, quinidine, quinine, raloxifene, ranitidine, remifentanil, repaglinide, reserpine, ricobendazole, rifabutin, rifampin, rifapentine, rimantadine, risperidone, ritonavir, rizatriptan, ropinirole, rosiglitazone, roxatidine, roxithromycin, salbutamol, saquinavir, selegiline, sertraline, sibutramine, sildenafil, sparfloxacin, spiramycins, stavudine, sufentanil, sulconazole, sulphasalazine, sulpiride, sumatriptan, tacrine, tamoxifen, tamsulosin, temazepam, terazosin, terbinafine, terbutaline, terconazole, terfenadine, tetramisole, thiabendazole, thioguanine, thioridazine, tiagabine, ticlopidine, timolol, tinidazole, tioconazole, tirofiban, tizanidine, tolterodine, topotecan, toremifene, tramadol, trazodone, triamterene, triazolam, trifluoperazine, trimethoprim, trimipramine, tromethamine, tropicamide, trovafloxacin, vancomycin, venlafaxine, vigabatrin, vinblastine, vincristine, vinorelbine, vitamin K₅, vitamin K₆, vitamin K₇, zafirlukast, zolmitriptan, zolpidem and zopiclone. Of course, any of the foregoing therapeutic agents may be included in the coating composition, as discussed previously, and any of the therapeutic agents discussed with regard the coating composition may alternatively be included in the core material.

The core material may be designed for delivering therapeutic agents intended to be delivered over a sustained period of time. The following are representative of such therapeutic agents: anti-inflammatory, antipyretic anti-spasmodics or analgesics such as indomethacin, diclofenac, diclofenac sodium, codeine, ibuprofen, phenylbutazone, oxyphenbutazone, mepirizole, aspirin, ethenzamide, acetaminophen, aminopyrine, phenacetin, butylscopolamine bromide, morphine, etomidoline, pentazocine, fenoprofen calcium, naproxen, selecxip, valdecxip, and tolamadol, anti-rheumatism drugs such as etodolac, anti-tuberculoses drugs such as isoniazide and ethambutol hydrochloride, cardiovascular drugs such as isosorbide dinitrate, nitroglycerin, nifedipine, barnidipine hydrochloride, nicardipine hydrochloride, dipyridamole, amrinone, indenolol hydrochloride, hydralazine hydrochloride, methyldopa, furosemide, spironolactone, guanethidine nitrate, reserpine, amosulalol hydrochloride, lisinopril, metoprolol, pilocarpine, and talcetin, antipsychotic drugs such as chlorpromazine hydrochloride, amitriptyline hydrochloride, nemonapride, haloperidol, moperone hydrochloride, perphenazine, diazepam, lorazepam, chlorodiazepoxide, adinazolam, alprazolam, methylphenidate, myrnasipran, peroxetin, risperidone, and sodium valproate, anti-emetics such as metoclopramide, lamocetron hydrochloride, granisetron hydrochloride, ondansetron hydrochloride, and azacetron hydrochloride, antihistamines such as chlorpheniramine maleate and diphenhydramine hydrochloride, vitamins suh as thiamine nitrate, tocopherol acetate, cycothiamine, pyridoxal phosphate, cobarnamide, ascortic acid, and nicotinamide, anti-gout drugs such as allopurinol, colchicine, and probenecide, anti-Parkinson's disease drugs such as levodopa and selegrine, sedatives and hypnotics such as amobarbital, bromuralyl urea, midazolam, and chloral hydrate, antineoplastics such as fluorouracil, carmofur, acralvidine hydrochloride, cyclophosphamide, and thiodepa, anti-allergy drugs such as pseudoephedrine and terfenadine, decongestants such as phenylpropanolamine and ephedorine, diabetes mellitus drugs such as acetohexamide, insulin, tolbutamide, desmopressin, and glipizide, diuretics such as hydrochlorothiazide, polythiazide, and triamterene, bronchodilatos such as aminophylline, formoterol fumarate, and theophylline, antitussives such as codeine phosphate, noscapine, dimorfan phosphate, and dextromethorphan, anti-arrhythmics, such as quinidine nitrate, digitoxin, propafenone hydrochloride, and procainamide, topical anesthetics such as ethyl aminobenzoate, lidocaine, and dibucaine hydrochloride, anti-convulsants such as phenytoin, ethosuximide, and primidone, synthetic glucocorticoids such as hydrocortisone, prednisolone, triamcinolone, and betamethasone, antiulceratives such as famotidine, ranitidine hydrochloride, cimetidine, sucralfate, sulpiride, teprenone, plaunotol, 5-aminosalicylic acid, sulfasalazine, omeprazole, and lansoprazol, central nervous system drugs, such as indeloxazine, idebenone, thiapride hydrochloride, bifemelane hydrocide, and calcium homopantothenate, antihyperlipoproteinemics such as pravastatin sodium, simvastatin, lovastatin, and atorvastatin, antibiotics such as ampicillin hydrochloride, phthalylsulfacetamide, cefotetan, and josamycin, BPH therapeutic agents such as tamsulosin hydrochloride, doxazosin mesylate, and terazosin hydrochloride, drugs affecting uterine motility such as branylcast, zafylcast, albuterol, ambroxol, budesonide, and reproterol, peripheral circulation improvers of prostaglandin I derivatives such as beraprost sodium, anticoagulants, hypotensives, agents for treatment of cardiac insufficiency, agents used to treat the various complications of diabetes, peptic ulcer therapeutic agents, skin ulcer therapeutic agents, agents used to treat hyperlipemia, tocolytics, etc. The therapeutic agent can be used in its free form or as a pharmaceutically acceptable salt. Moreover, one or a combination of two or more therapeutic agents may be present in the core material.

In some embodiments, the therapeutic agent in the core material includes conjugated estrogens. "Conjugated estrogens" (CE) as used herein includes both natural and synthetic conjugated estrogens, such as the compounds described in the United States Pharmacopia (USP 23), as well as other estrogens so considered by those skilled in the art. Further, "conjugated estrogens" refers to esters of such compounds, such as the sulfate esters, salts of such compounds, such as sodium salts, and esters of the salts of such compounds, such as sodium salts of a sulfate ester, as well as other derivatives known in the art. Some specific examples include: 17-alpha and beta-dihydroequilin, equilenin, 17-alpha and beta-dihydroequilenin, estrone, 17-beta-estradiol, and their sodium sulfate esters.

Although CE are typically a mixture of estrogenic components, such as estrone and equilin, the core material may be formulated to either utilize such a mixture, or to include only selected or individual estrogenic components. These CE may be of synthetic or natural origin. Examples of synthetically produced estrogens include, *inter alia,* sodium estrone sulfate, sodium equilin sulfate, sodium 17α-dihydroequilin sulfate, sodium 17β-dihydroequilin sulfate, sodium 17α-estradiol sulfate, sodium 17β-estradiol sulfate, sodium equilenin sulfate, sodium 17α-dihydroequilenin sulfate, sodium 17β-dihydroequilenin sulfate, estropipate and ethinyl estradiol. The alkali metal salts of 8,9-dehydroestrone and the alkali metal salts of 8,9-dehydroestrone sulfate ester, as described in U.S. Patent No. 5,210,081, also may be used. Naturally occurring CE are usually obtained from pregnant mare urine and then are processed and may be stabilized. Examples of such processes are set forth in U.S. Pat. Nos. 2,565,115 and 2,720,483.

Many CE products are now commercially available. Preferred among these is the naturally occurring CE product known as Premarin® (Wyeth, Madison, NJ). Another commercially available CE product prepared from synthetic estrogens is Cenestin® (Duramed Pharmaceuticals, Inc., Cincinnati, Ohio). The specific CE dose included in the core material may be any dosage required to achieve a specific therapeutic effect, and may vary depending on the specific treatment indicated, and on the specific CE included in the tablet. However, in general, dosages of CE included in the tablet can range from about 0.1 mg CE/dosage form to about 5.0 mg CE/dosage form, with dosages of from about 0.3 mg CE/dosage form to about 2 mg CE/dosage form preferred. In some embodiments, the dosage of CE is from about 0.3 mg CE/dosage form, about 0.45 mg CE/dosage form, about 0.625 mg CE/dosage form, about 0.9 mg CE/dosage form, or about 1.25 mg CE/dosage form. Viewed alternatively, based on the total weight of the solid dosage form, on a dry weight basis, the amount of CE/dosage form may range from about 0.05 weight % to about 1.0 weight %, with amounts of from 0.1 weight % to about 0.3 weight % preferred.

Thus, in addition to the coating compositions described previously, the present invention is also directed to solid dosage forms comprising a core material and one or more coating disposed thereon, as each element has been described heretofore. In certain embodiments, the solid dosage forms comprise from about 30 weight % to about 70 weight % of the core material, and from about 30 weight % to about 70 weight % of the coating, and in still further embodiments, from about 40 weight % to about 60 weight % of the core material and from about 40 weight % to about 60 weight % of the coating. The solid dosage forms also can optionally include one or more additional coats. For example, a further sugar coating as described herein, disposed either on top of the coating previously described, or in between the core and coating. The solid dosage form also can include a color coat and/or a polish coat. In some embodiments, the color coat constitutes from about 0.5 weight % to about 10 weight % of the dosage form, and/or a polish coat, that constitutes from about 0.1 weight % to about 5 weight % of the dosage form.

In preferred embodiments, the solid dosage form is in the form of a coated tablet, wherein the core material comprises at least one therapeutic agent and at least one pharmaceutically acceptable excipient. In some embodiments, the excipient in the core material can include one or more cellulosic materials in a cumulative amount of from about 15 weight % to about 50 weight % of the solid dosage form; from about 18 weight % to about 40 weight % of the solid dosage form; or from about 40 weight % to about 45 weight % of the solid dosage form. In some embodiments, the cellulosic material includes Hypromellose, microcrystalline cellulose, and sodium carboxymethylcellulose, and mixtures thereof.

In addition to the cellulosic material, in certain embodiments, the core material also includes a sugar excipient, such as lactose. A preferred form of lactose is lactose monohydrate, which is well known to those of skill in the art. In some embodiments, the dosage form of the invention is a coated tablet that comprises a tablet core containing from about 4 weight % to about 35 weight % lactose monohydrate, and from about 20 weight % to about 40 weight % cellulosic material, based on the total weight of the solid dosage form. In a further such embodiment, the cellulosic material is a mixture of microcrystalline cellulose and Hypromellose. A lubricant, such as, for example, magnesium stearate, also can be utilized in the tablet core of such embodiments, typically, at an amount of up to about 1 weight %, based on the total weight of the solid dosage form.

This formulations of the invention can be utilized with any of the aforementioned therapeutic agents, but is particularly suited for coated tablets that utilize conjugated estrogens, such as the conjugated estrogens desiccation with lactose. In some embodiments, from about 3 weight % to about 15 weight % of the conjugated estrogens dessication with lactose (about 4.3 weight % conjugated estrogens), based on the total weight of the solid dosage form, is included in the tablet core. In still further embodiments, about 4 weight % to about 10 weight % of the conjugated estrogens desiccation with lactose (about 4.3 weight % conjugated estrogens), is included in the tablet core. In certain embodiments, the conjugated estrogens are present in an amount from about 0.1 mg CE/dosage form to about 5.0 mg CE/dosage form, or from about 0.3 mg CE/dosage form to about 2 mg CE/dosage form. In some embodiments the dosage of CE is from about 0.3 mg CE/dosage form, about 0.45 mg CE/dosage form, about 0.625 mg CE/dosage form, about 0.9 mg CE/dosage form, or about 1.25 mg CE/dosage form. Viewed alternatively, in such embodiments, the amount of CE/dosage form, based on the total weight of the solid dosage form (on a dry weight basis), is present from about 0.05% weight % to about 1.0 weight %, or further, from 0.1 weight % to about 0.3 weight %. The tablet core may then be coated with the coating composition described above, in an amount of from about 30 weight % to about 75 weight %, based on the total weight of the solid dosage form, to produce a coated tablet. An optional color coat and/or polish coat may also be applied, as described previously.

In certain embodiments, the solid dosage form comprises a core material and at least one coating, wherein the core material includes (based on the total weight of the solid dosage form on a dry weight basis) from about 0.1 weight % to about 0.3 weight % conjugated estrogens; from about 4 weight % to about 35 weight % of at least one sugar, for example lactose monohydrate; from about 5 weight % to about 10 weight % of at least one binder, for example microcrystalline cellulose; from about 10 weight % to about 35 weight % of at least one water soluble polymer, for example, hydroxypropyl methylcellulose; and from about 0 to about 1 weight % of at least one lubricant, for example, magnesium stearate. In some such embodiments, the solid dosage form further comprises a color coat, a polish coat, or both a color coat and a polish coat.

One of the primary advantages of the present invention is that a wide variety of tablet cores, prepared according to the various processes known in the art, can readily be coated with the coating composition of the present invention, utilizing the simple spray techniques more frequently associated with film-coating applications. Thus, in a further embodiment, the present invention is directed to processes that comprise providing a tablet core and applying, e.g. by spraying, onto the core a sugar coating composition, as previously described. The invention is also directed to the products of such process, e.g. a coated tablet core. It should be noted, however, that although utilizing such spray techniques provides certain advantages; it is by no means required that the composition be applied in this manner. Other methods for coating pharmaceutical dosage forms, such as, for example, use of a fluidized bed application process, are well known to those of ordinary skill in the art.

In certain embodiments, the sugar coating composition is disposed directly onto the tablet core, without the need for intervening sealing layers, as are used typically in traditional sugar-coating methods. If desired, however, a sealing layer, such as shellac and other agents known to those in the art, may be applied to the tablet core prior to application of the sugar coating composition. In some embodiments, the sugar coating composition, which contains medroxyprogesterone acetate, as previously described, is disposed directly onto the tablet core without a non-therapeutic agent containing sugar coat being first applied, or an intervening sealing layer.

In some embodiments, the sugar coating composition is applied in a ratio of sugar coating to tablet core of from 3:1 to about 1:3, or in a ratio from about 2:1 to about 1:2, or in a ratio of from about 1.25:1 to about 1:1.25; and thus, providing a coated tablet. In some embodiments, the sugar coating composition is sprayed at an air flow rate of from about 500 cubic feet per minute to about 9000 cubic feet per minute; and further, at an air flow rate of from about 1000 cubic feet per minute to about 5000 cubic feet per minute. In some embodiments, the tablet core temperature is about 35°C to about 50°C, while the inlet temperature is about 50°C to about 80°C. While the processes of the present invention further may include the steps of spraying a color coat and/or polish coat onto the sugar coat, such steps are optional, and all of the coating steps may be carried out in a single coating pan. Also, the step of printing a logo, trademark, word, symbol or the like optionally may be included in the processes of the present invention. Printing may be performed by any of the methods well known to those skilled in the art.

Thus, the process of the present invention may comprise the steps of providing tablet cores, placing the tablet cores into a coating pan, such as a perforated coating pan commonly utilized in film-coating applications such as a perforated pan with side-vents, then sequentially spraying the tablet cores with the sugar coating composition, the color coat, and the polish coat (if desired). Spray techniques for coating tablets are well known to those of skill in the art, and are described, for example, in Stuart C. Porter, Coating of Pharmaceutical Dosage Forms, Remington: The Science and Practice of Pharmacy, 20th Ed., Chap. 46, Alfonso R. Gennaro, ed., Philadelphia College of Pharmacy and Science, Philadelphia, PA (2000).

The processes of the present invention are much simpler, less labor intensive, and less reliant upon operator expertise than the traditional sugar-coating techniques known in the prior art. Additionally, due to the unique combination of ingredients utilized in the sugar coating composition and the volume of sugar coating that is applied, the coated tablets produced by the processes of the present invention are remarkably hard, durable, and resistant to cracking, even when highly hygroscopic tablet cores are utilized. In some embodiments, a plurality of tablet cores coated with the sugar coating compositions of the present invention contain cracking in less than 6 percent of the coated tablet cores. In further such embodiments, the percentage of cracks is about 1 to about 5 percent; and in still further embodiments, less than 1 percent. Additionally, the coating provides an excellent barrier to prevent the release of odors from the tablet core, and to prevent atmospheric elements from reaching and degrading the therapeutic agent(s) in the tablet core. Thus, the coating compositions and processes described herein are particularly well suited for preparing solid dosage forms that utilize therapeutic agents or other materials having strong odors, such as, sulfur-containing compounds, in the core material.

Moreover, as discussed previously, the sugar coating composition includes one or more therapeutic agents, in addition to the therapeutic agent(s) that are present within the tablet core. Thus, therapeutic agents can be separated by their compartmentalization into either the core or the coating, to thereby minimize unwanted chemical interaction between the agents. Additionally, since the coating and tablet core can be designed to release their ingredients at different rates, the present invention may be utilized to provide, in a single dosage form, both a quick release and a sustained release formulation of the therapeutic agent(s). Also, it has been found unexpectedly that utilizing, in a sugar coating containing a therapeutic agent, a control release agent, which has been used traditionally for controlling drug diffusion from a therapeutic core, allows effective control of the therapeutic agent in the sugar coating itself. Thus, the second polymer of the sugar coating compositions of the inventions functions as the aforementioned control release agent and includes a polymethacrylate that will function to retard the release of the active therapeutic agent from the coating. In some preferred embodiments, the second polymer, which is water soluble or water dispersible is Eudragit® NE30D. The features of the coating compositions of the present invention, as described above, are particularly useful when delivering certain therapeutic agents such as hormones, where, for example, a quick release of one hormone, located in the coating, may be followed by a slow, sustained release of a second hormone from the tablet core. Of course, this property of the solid dosage forms may also be utilized to provide both a quick release and a sustained release of a single therapeutic agent, where both the tablet core and the coating contain the same therapeutic agent.

### EXAMPLES

The invention is further demonstrated in the following examples. The examples are for purposes of illustration and are not intended to limit the scope of the present invention.

### EXAMPLE 1

**PREPARATION OF 1.25 MG CONJUGATED ESTROGEN COATED TABLETS**

| **Tablet Core** | **Amt/tablet (mg)** |
|---|---|
| CE Desiccation with Lactose @ 42.9 mg/g | 20.14 |
| Lactose Monohydrate, NF (Spray Dried) | 120.3 |
| Microcrystalline Cellulose, NF | 36.0 |
| Hypromellose, USP, 2208, K100M (100,000 cps) | 54.0 |
| Magnesium Stearate, NF | 0.600 |
| Totals | 240.0 |

| **Sugar Coat Filler Suspension (A)** | |
|---|---|
| Hydroxypropyl Cellulose, NF | 13.80 |
| Hypromellose, USP, 2910, E5 (5 cps) | 59.8 |
| Hypromellose, USP, 2910, E15 (15 cps) | 15.00 |
| Microcrystalline Cellulose, NF | 18.40 |
| Polyethylene Glycol 400, NF | 8.05 |
| Sucrose, NF | 115.0 |
| Totals | 230 |

| **Color Suspension (B)** | |
|---|---|
| Opadry^{®} II, Yellow, 40L12916 | 15.00 |

| **Polish Solution (C)** | |
|---|---|
| Opaglos^{®} 2, Clear, 98Z19173 | 10.00 |
| Total Finished Tablet Weight | 495 |

| | |
|---|---|
| Tablet Core 1. Add the lactose monohydrate, NF, C.E. desiccation with lactose, microcrystalline cellulose, NF, and the Hypromellose, USP, 2208 (K100M Premium, CR) to a high shear mixer. Blend all ingredients with plows only. 2. Granulate the blend with water, U.S.P., purified mixing with plows and choppers. 3. Size the granulation using a cutting mill. 4. Dry the granulation in a fluid bed dryer. 5. Size the dried granulation using a cutting mill. 6. Transfer the granulation to a V Blender. Blend. 7. Add the magnesium stearate, NF to the V Blender in step #6. Blend. 8. Compress granulation to target tablet weight, hardness and thickness using appropriately sized oval tooling on a rotary tablet press. | |

Similar formulations and manufacturing procedures have been developed and prepared for tablets containing CE at strengths of 0.3 mg, 0.45 mg, 0.625 mg and 0.9 mg.

### Sugar Coat Filler Suspension (A)

1. Place the Purified Water in a suitable stainless steel container.
2. With mixing add the hydroxypropyl cellulose, hydroxypropyl methylcellulose, 2910, E5, hydroxypropyl methylcellulose, 2910, E15, and the polyethylene glycol 400 to Step #1.
3. With mixing, add the sucrose to Step #2 and mix until all ingredients are dissolved.
4. With mixing, add the microcrystalline cellulose to Step #3.
5. Allow Step #4 to de-aerate, bring to theoretical weight with purified water with mixing, and cool to room temperature prior to use, if necessary.
6. Continue mixing Step #5 until the Filler application is complete.

### Color Suspension (B)

1. Add the purified water to a suitable stainless steel container.
2. With mixing, add the Opadry^{®} II Yellow (Colorcon, West Point, PA) to Step #1. Continue stirring until the suspension is complete.
3. Bring Step #2 to theoretical weight with purified water, with mixing, if necessary.
4. Continue mixing Step #3 until the color application is complete.

### Polish Solution (C)

1. Add the Purified Water to a suitable stainless steel container.
2. With mixing, add the Opaglos® 2 Clear (Colorcon, West Point, PA) to Step #1. Continue mixing until the solution is complete.

Prior to application, allow the solution to de-aerate and bring Step #2 to theoretical weight with Purified Water with mixing, if necessary. Mixing is not necessary during the application.

### Tablet Coating

1. Load the compressed tablet cores into perforated coating pan(s).
2. Apply sufficient sugar coat filler suspension (A) to step #1 tablet cores to achieve desired coating weight gain above the average tablet core weight.
3. Apply sufficient color suspension (B) to step #2 filled tablets to achieve desired coating weight gain above the average filled tablet weight.
4. Apply sufficient polish solution (C) to step #3 colored tablets to achieve desired coating weight gain above the average colored tablet weight.

### EXAMPLE 2

The tablet core composition utilized in Example 1 contains hydrogel (Hypromellose) type polymers which are useful to modify/control the release of the active ingredient. This type of tablet core is flexible, and prone to swelling, however. A conventional sugar coat tends to be brittle and is prone to chipping, cracking and splitting due to processing conditions and/or if exposed to inappropriate mechanical stress *(*Pharmaceutical Coating Technology, Cole E, Hogan J., Aulton M., 1995, page 62, section 3.5). This example shows the ability of the coating composition of the present invention to resist cracking.

As controls, tablets containing hydrogel polymers and 1.25 mg/tablet of water-soluble estrogens, similar to the tablet cores described in Example 1, were coated with a conventional sugar coat. When exaggerated physical abuse was applied to the coated tablets, the vast majority of the tablets developed cracks in the coating. When the same tablets were coated with a sugar coat composition of the present invention, similar to the sugar coating suspension utilized in Example 1, and achieving the same coating weight gain, no cracks were observed in the coating. These data are presented in Table 1. Also included are data on traditional tablet cores (non-swelling) coated with a conventional sugar coat. These data also show that some coat cracking can be observed even on these more stable tablet cores with a conventional sugar coat.

**Table 1**

| **NEW TABLET FORMULATION** | | | |
|---|---|---|---|
| **Coated Tablet Cracking with Exaggerated Physical Abuse** | | | |
| Formulation | Number of Batches Tested | Total # of tablets tested | Average % Cracks |
| Hydrogel core and sugar coating composition of the present invention | 7 | 7x100=700 | 0 |
| Traditional core and conventional Sugar Coat | 5 | 5x 100=500 | 14 |
| Hydrogel core and conventional Sugar Coat | 7 | 7x100=700 | 95 |

### EXAMPLE 3

### PREPARATION OF 0.45 MG CE/1.5 MG MPA COATED TABLETS WITH INTERVENING SUGAR COAT

In this example, 0.45 mg CE tablet cores were prepared and coated with a sugar coat suspension, in accordance with the formulation and manufacturing process of Example 1, except that the tablet core weight is 120 mg and the total solids filler sugar coat applied was 90 mg. An active filler suspension containing medroxyprogesterone acetate (MPA) was then applied followed by the color and polish coats, as described below. Alternatively, the active filler suspension could be sprayed directly onto the tablet cores, without the intervening coating step (e.g., a first sugar coating of the present invention such as Example 4, below).

**0.45 mg/tablet CE/1.50 mg/tablet MPA Preparation**

| | **Input/Dosage Unit** | |
|---|---|---|
| **Ingredient** | **Input** | **Unit** |
| **Sugar Coated Core** | 210 | mg |

| **Active MPA Filler Suspension Overcoat (D)** | | |
|---|---|---|
| Medroxyprogesterone Acetate, USP | 1.5 | mg |
| Sucrose, NF | 50.5 | mg |
| Microcrystalline Cellulose, NF | 8.31 | mg |
| Hydroxypropyl Cellulose, NF | 6.23 | mg |
| Hypromellose, 2910, USP, E6 (6 cps) | 27.1 | mg |
| Hypromellose, 2910, USP, E15 (15 cps) | 6.75 | mg |
| Polyethylene Glycol 400, NF | 3.63 | mg |
| Purified Water, USP * | 474 | mg |
| | | |

| **Color Suspension (E)** | | |
|---|---|---|
| Hypromellose, 2910, USP, E3 (3 cps) | 7.79 | mg |
| Polyethylene Glycol 400, NF | 0.780 | mg |
| Titanium Dioxide, USP | 2.86 | mg |
| Ferric Oxide, NF, Yellow | 0.571 | mg |
| Purified Water, USP * | 69.6 | mg |
| | | |

| **Polish Suspension (F)** | | |
|---|---|---|
| Opaglos^{®} 2, Clear, 97W19196 | 6.0 | mg |
| Purified Water, USP * | 114 | mg |
| **Total tablet weight** | 332 | mg |

| | | |
|---|---|---|
| * Removed during processing. | | |

The tablet core and sugar coat filler suspension are prepared substantially as set forth in Example 1.

### Active MPA Filler Suspension (D)

1. Place the Purified Water in a suitable stainless steel container.
2. With mixing add the MPA powder until uniformly dispersed.
3. With mixing add the Hypromellose, 2910, E6, Hypromellose, 2910, E15, hydroxypropyl cellulose, and the polyethylene glycol 400 and sucrose; mix until all ingredients are dissolved.
4. Allow suspension to de-aerate.
5. With mixing add the microcrystalline cellulose.
6. Bring the suspension to theoretical weight with purified water with mixing, and continue mixing until application is complete.

### Color Suspension (E)

1. Place a portion of purified water to a suitable stainless steel container.
2. With mixing, add the ferric oxide and titanium dioxide; blend until uniform.
3. Add the balance of the purified water to a suitable stainless steel container.
4. With mixing, add the Hypromellose, E3 and PEG 400 and stir until dissolved.
5. Add the Step #2 color mixture and continue stirring until the suspension is complete.
6. Allow suspension to de-aerate and bring to theoretical weight with purified water.
   Continue mixing until the color application is complete.

### Polish Solution (F)

1. Add the Purified Water to a suitable stainless steel container.
2. With mixing, add the Opaglos^{®} 2 Clear. Continue mixing until the solution is complete. Allow the solution to de-aerate and bring to theoretical weight with purified water with mixing, if necessary.

### Tablet Coating

1. Load the compressed tablet cores into perforated coating pan(s).
2. Apply sufficient sugar coat filler suspension (A) to Step #1 tablet cores to achieve an average filler solids weight of 90 mg (± 5 mg) above the average compressed core weight.
3. Apply sufficient active filler suspension (D) to Step #2 tablet cores to achieve an average filler solids weight of 104 mg (± 5 mg) above the average inert filled tablet core weight.
4. Apply sufficient color suspension (E) to Step #3 filled tablets to achieve an average color solids weight of 12 mg (± 2 mg) above the average MPA filled tablet weight.
5. Apply sufficient polish solution (F) to Step #4 colored tablets to achieve an average total polish solids weight of 6 mg (± 1 mg) above the average colored tablet weight.

The utility of the 0.45mg/1.5mg CE/MPA tablet is further supported based on actual *in vivo* data from a pilot bioavailability study in dogs. Figure 1 shows plasma MPA levels in 6 beagle dogs following oral administration of the subject tablet formulation. This data confirms that this type of formulation is capable of providing reproducible blood levels and is a viable delivery system for this class of drug.

### EXAMPLE 4

### PREPARATION OF 0.45 MG CE/1.5 MG MPA COATED TABLETS - I MPA-CONTAINING COAT APPLIED DIRECTLY

In this example, 0.45 mg CE tablet cores were prepared and coated with an active filler coat suspension, in accordance manufacturing process of Example 3, except the active filler coat suspension was sprayed directly onto the tablet core. That is to say, the coated tablet contained one sugar coat, which contained the MPA, and no intervening active-free sugar coat or sealing later were applied initially to the tablet core.

**0.45 mg/tablet CE/1.50 mg/tablet MPA Preparation**

| **Ingredient** | **Input/Dosage Unit** | |
|---|---|---|
| | **Input** | **Unit** |
| **Tablet Core** | 120 | mg |
| CE Dessication with Lactose @ 42.9 mg/g | 10.5 | mg |
| Lactose Monohydrate, NF (Spray Dried) | 58.2 | mg |
| Microcrystalline Cellulose, NF, EP | 18.0 | mg |
| Hypromellose, USP, 2208, K100M (100,000 cps) | 33.0 | mg |
| Magnesium Stearate, NF, EP | 0.300 | mg |
| | | |
| **Active MPA Filler Suspension Overcoat (G)** | | |
| Medroxyprogesterone Acetate, USP | 1.5 | mg |
| Sucrose, NF | 36.5 | mg |
| Microcrystalline Cellulose, NF, EP | 6.09 | mg |
| Hydroxypropyl Cellulose, Klucel^{®} EF Pharma, NF | 4.57 | mg |
| Hypromellose, 2910, USP, E6 | 19.8 | mg |
| Hypromellose, 2910, USP, E15 | 4.95 | mg |
| Polyethylene Glycol 400, NF | 2.66 | mg |
| Polyacrylate dispersion 30% (Eudragit^{®} NE30D), EP | 46.5* | mg |
| Purified water, USP** | 300 | mg |
| | | |
| **Color Suspension (H)** | | |
| Hypromellose, 2910, USP, E6 | 3.40 | mg |
| Polyethylene Glycol 400, NF | 0.340 | mg |
| Titanium Dioxide, USP | 1.07 | mg |
| Ferric Oxide, NF, Yellow | 0.187 | mg |
| Purified Water, USP** | 30.7 | mg |
| | | |
| **Polish Suspension (I)** | | |
| Opaglos^{®} 2, Clear, 98Z19173 | 2.0 | mg |
| Purified Water, USP** | 38 | mg |
| **Total tablet weight** | 217 | mg |

| | | |
|---|---|---|
| * 13.9 mg solids ** Removed during processing | | |

The tablet core was prepared substantially as set forth in Example 1.

### Active MPA Filler Suspension (G)

1. Place the purified water in a suitable stainless steel container.
2. With mixing, add the MPA powder until uniformly dispersed.
3. With mixing add the Hypromelloses, hydroxypropyl cellulose, polyethylene glycol 400 and sucrose; mix until all ingredients are uniformly dispersed.
4. With mixing, add the microcrystalline cellulose until uniformly dispersed. Cool the suspension to room temperature.
5. With mixing add the polyacrylate dispersion 30% (Eudragit^{®} NE 30 D) EP through a stainless steel screen. Mix until uniformly dispersed.
6. Bring the suspension of Step #5 to theoretical weight with purified water with mixing, if necessary.
7. Continue mixing until the MPA filler application is complete.

### Color Suspension (H)

1. Place the purified water to a suitable stainless steel container.
2. With mixing, add the ferric oxide and titanium dioxide; blend until uniform.
3. Add the Hypromellose, 2910, USP, E6 and PEG 400; continue mixing until the suspension is complete.
4. Allow the suspension of Step #3 to de-aerate; and bring the suspension to theoretical weight with purified water with mixing, if necessary.
5. Continue mixing until the color application is complete.

### Polish Solution (I)

1. Add the Purified Water to a suitable stainless steel container.
2. With mixing, add the Opaglos^{®} 2 Clear. Continue mixing until the solution is complete.
3. Prior to application, allow the solution to de-aerate and bring to theoretical weight with purified water with mixing, if necessary.

### Tablet Coating

1. Load the tablet cores into perforated coating pan(s).
2. Apply sufficient active filler suspension (G) to Step #1 tablet cores to achieve an average filler solids weight of 90 mg (± 2 mg) above the average tablet core weight.
3. Cure the tablets at a tablet bed temperature of 45-50 °C for approximately 1 hour.
4. Apply sufficient color suspension (H) to Step #3 MPA filled tablets to achieve an average color solids weight of 5 mg (± 1 mg) above the average MPA filled tablet weight.
5. Apply sufficient polish solution (I) to Step #4 colored tablets to achieve an average total polish solids weight of 2 mg (± 1 mg) above the average colored tablet weight.

Similar formulations that have an average sugar fill coat containing MPA in an amount of from about 0.5 to 10 mg can be prepared as described in any of the preceding Examples.

### EXAMPLE 5

The following prophetic example describes the production of coated tablets of the present invention containing a blend of the synthetic conjugated estrogenic substances sodium estrone sulfate, sodium equilin sulfate, sodium 17α-dihydroequilin sulfate, sodium 17β-dihydroequilin sulfate, sodium 17α-estradiol sulfate, sodium 17β-estradiol sulfate, sodium equilenin sulfate, sodium 17α-dihydroequilenin sulfate, sodium 17β-dihydroequilenin sulfate. Utilizing procedures widely known to those of ordinary skill in the art, the conjugated estrogenic substances are mixed with commonly used excipients and compressed to form tablet cores of various strengths containing, for example, 0.3 mg, 0.45 mg, 0.625 mg, 0.9 mg, and 1.25 mg CE. The tablet cores then may be coated as described in any of the preceding examples.

### EXAMPLE 6

### DISSOLUTION STUDY OF 0.45 mg/TABLET CE/1.50 mg/TABLET MPA PREPARATIONS

Since the active filler sugar coating and tablet core can be designed to release their ingredients at different rates, the present invention may be utilized to provide, in a single dosage form, both a quick release and a sustained release formulation of the therapeutic agent(s). The release time from the coating can be affected by, for example, the total solids of active applied to the core, the thickness of the active filler sugar coat applied, and the presence of a release controlling agent. This Example measured the MPA dissolution profiles of three formulations of 0.45 mg/tablet CE/1.50 mg/tablet MPA. The preparations were prepared as described in the preceding examples. All three preparations had the MPA filler sugar coat applied directly onto the CE tablet cores. The MPA filler sugar coat of one formulation further contained a second polymer, as described above, *viz.* polymethacrylate (15% Eudragit^{®} NE30D), and the total solids of MPA sugar coat applied was 90 mg such as that of Example 4. The MPA sugar coat of the other two formulations were similar in composition to the Active Sugar Filler Suspension (D) and (S) of Example 3 and 11, respectively, i.e., with one water soluble polymer, with the total solids of MPA sugar coat applied being 90 mg and 200 mg, respectively. All three formulations were polished.

**Percent MPA Dissolved^{#}**

| **Time (min.)** | **1.5 mg MPA + 15% NE30D @ 90 mg** | **1.5 mg MPA @ 200 mg** | **1.5 mg MPA @ 90 mg** |
|---|---|---|---|
| 15 | 2 | 10 | 12 |
| 30 | 4 | 27 | 42 |
| 45 | 7 | 44 | 67 |
| 60 | 10 | 60 | 83 |

| | | | |
|---|---|---|---|
| ^{#} Dissolution was performed at 50 rpm, in 0.54% SLS, 0.02 M sodium acetate, pH 4.5 in standard vessels with sinkers; n=6. | | | |

The data confirms that a coating containing a second polymer, which traditionally has been used to delay release of therapeutic agents from a tablet core, can effectively delay the release of a therapeutic agent from the coating applied to a tablet core. The data also confirms that a greater amount of active filler sugar coat applied to the tablet core effectively can slow the release of the therapeutic agent compared to a lesser amount of active filler sugar coat similarly applied to the tablet core.

Examples 7-10 show the compositions of coated tablets of the invention containing 0.3 mg, 0.45 mg, 0.625 mg, 0.9 mg of conjugated estrogens, respectively. The tablet core and sugar coat filler suspension are prepared substantially as set forth in any of the preceding Examples.

### EXAMPLE 7

**COMPOSITION OF 0.3 CONJUGATED ESTROGEN COATED TABLET**

| **Tablet Core** | **Amt/tablet (mg)** |
|---|---|
| CE Desiccation with Lactose @ 4.29 % CE | 6.993 |
| Lactose Monohydrate (Spray Dried) | 61.7 |
| Microcrystalline Cellulose | 18.0 |
| Hypromellose 2208, K100M (100,000 cps) | 33.0 |
| Magnesium Stearate | 0.300 |
| Totals | 120 mg |

| **Sugar Coat Filler Suspension (J)** | |
|---|---|
| Sucrose | 45.0 |
| Microcrystalline Cellulose | 7.20 |
| Hydroxypropyl Cellulose | 5.40 |
| Hypromellose, 2910, E6 (6 cps) | 23.4 |
| Hypromellose, 2910, E15 (15 cps) | 5.85 |
| Polyethylene Glycol 400 | 3.15 |
| Totals | 90 |

| **Color Suspension (K)** | |
|---|---|
| Opadry^{®} Green | 5.00 |

| **Polish Solution (L)** | |
|---|---|
| Opaglos^{®} 2, Clear | 3.00 |
| Total Finished Tablet Weight | 218 |

### EXAMPLE 8

**COMPOSITION OF 0.45 MG CONJUGATED ESTROGEN COATED TABLETS**

| **Tablet Core** | **Amt/tabtet (mg)** |
|---|---|
| CE Desiccation with Lactose @ 4.29 % CE | 10.4895 |
| Lactose Monohydrate (Spray Dried) | 58.2 |
| Microcrystalline Cellulose | 18.0 |
| Hypromellose 2208, K100M (100,000 cps) | 33.0 |
| Magnesium Stearate | 0.300 |
| Totals | 120 mg |

| **Sugar Coat Filler Suspension (M)** | |
|---|---|
| Sucrose | 45.0 |
| Microcrystalline Cellulose | 7.20 |
| Hydroxypropyl Cellulose | 5.40 |
| Hypromellose, 2910, E6 (6 cps) | 23.4 |
| Hypromellose, 2910, E15 (15 cps) | 5.85 |
| Polyethylene Glycol 400 | 3.15 |
| Totals | 90 |

| **Color Suspension (N)** | |
|---|---|
| Opadry^{®} Blue | 5.00 |

| **Polish Solution (O)** | |
|---|---|
| Opaglos^{®} 2, Clear | 3.00 |
| Total Finished Tablet Weight | 218 |

### EXAMPLE 9

**COMPOSITION OF 0.635 MG CONJUGATED ESTROGEN COATED TABLETS**

| **Tablet Core** | **Amt/tablet (mg)** |
|---|---|
| CE Desiccation with Lactose @ 4.29 % CE | 14.5688 |
| Lactose Monohydrate (Spray Dried) | 54.1 |
| Microcrystalline Cellulose | 18.0 |
| Hypromellose 2208, K100M (100,000 cps) | 33.0 |
| Magnesium Stearate | 0.300 |
| Totals | 120 mg |

| **Sugar Coat Filler Suspension (P)** | |
|---|---|
| Sucrose | 45.0 |
| Microcrystalline Cellulose | 7.20 |
| Hydroxypropyl Cellulose | 5.40 |
| Hypromellose, 2910, E6 (6 cps) | 23.4 |
| Hypromellose, 2910, E15 (15 cps) | 5.85 |
| Polyethylene Glycol 400 | 3.15 |
| Totals | 90 |

| **Color Suspension (Q)** | |
|---|---|
| Opadry^{®} Maroon | 5.00 |

| **Polish Solution (R)** | |
|---|---|
| Opaglos^{®} 2, Clear | 3.00 |
| Total Finished Tablet Weight | 218 |

### EXAMPLE 10

**COMPOSITION OF 0.9 MG CONJUGATED ESTROGEN COATED TABLETS**

| **Tablet Core** | **Amt/tablet (mg)** |
|---|---|
| CE Desiccation with Lactose @ 4.29 % CE | 20.979 |
| Lactose Monohydrate (Spray Dried) | 78.0 |
| Microcrystalline Cellulose | 26.0 |
| Hypromellose 2208, K100M (100,000 cps) | 47.6 |
| Magnesium Stearate | 0.433 |
| Totals | 173 mg |

| **Sugar Coat Filler Suspension (S)** | |
|---|---|
| Sucrose | 65.0 |
| Microcrystalline Cellulose | 10.4 |
| Hydroxypropyl Cellulose | 7.80 |
| Hypromellose, 2910, E6 (6 cps) | 33.8 |
| Hypromellose, 2910, E15 (15 cps) | 8.45 |
| Polyethylene Glycol 400 | 4.55 |
| Totals | 130 |

| **Color Suspension (T)** | |
|---|---|
| Opadry^{®} White | 7.00 |

| **Polish Solution (U)** | |
|---|---|
| Opaglos^{®} 2, Clear | 3.00 |
| Total Finished Tablet Weight | 313 |

Example 11 shows the composition of a coated tablet of the invention containing 0.45 mg of conjugated estrogens, and 1.50 mg of MPA.

### EXAMPLE 11

**COMPOSITION OF 0.45 MG CE / 1.5 MG MPA COATED TABLETS - II MPA-CONTAINING COAT APPLIED DIRECTLY**

| **Tablet Core** | **Amt/tablet (mg)** |
|---|---|
| CE Desiccation with Lactose @ 4.29 % CE | 10.49 |
| Lactose Monohydrate, NF/EP (powder) | 58.21 |
| Microcrystalline Cellulose, NF/EP | 18.0 |
| Hypromellose, USP, 2208 (100,000 cps) (K100M Prem, CR) | 33.0 |
| Magnesium Stearate, NF/EP, Hyqual Vegetable Code | 0.300 |
| Totals | 120 mg |

| **Active MPA Filler Suspension Overcoat (S)** | |
|---|---|
| Medroxyprogesterone Acetate, USP/EP | 1.500 |
| Sucrose, NF | 98.50 |
| Microcrystalline Cellulose, NF/EP | 16.00 |
| Hydroxypropyl Cellulose, Klucel EF Pharma, NF | 12.00 |
| Hypromellose, 2910, USP/EP E6 | 52.00 |
| Hypromellose, 2910, USP E15 | 13.00 |
| Polyethylene Glycol 400, NF | 7.000 |
| Totals | 200 |

| **Color Suspension (T)** | |
|---|---|
| Spectrablend Yellow | 6.00 |

| **Polish Solution (U)** | |
|---|---|
| Opaglos^{®} 2, Clear | 1.50 |
| Total Finished Tablet Weight | 328 |

## Claims

1. A composition in the form of an aqueous suspension comprising water and a solids component that comprises:
from 30 weight percent to 60 weight percent of at least one sugar;
from 5 weight percent to 10 weight percent of at least one binder;
from 3 weight percent to 10 weight percent of at least one hydroxyalkyl cellulose;
from 15 weight percent to 50 weight percent of at least one water soluble polymer;
the therapeutic agent medroxyprogesterone acetate in an amount of up to 3 weight percent;
a second polymer that is water soluble or water dispersible, in an amount of up to 20 weight percent; and
optionally, at least one plasticizer in an amount of up to 8 weight percent;
wherein
the sugar comprises sucrose;
the binder comprises microcrystalline cellulose;
the hydroxyalkyl cellulose comprises hydroxypropyl cellulose;
the water soluble polymer comprises hydroxypropyl methylcellulose;
the second polymer comprises a polymethacrylate;
and the plasticizer, when present, comprises polyethylene glycol.

2. The composition of claim 1 that comprises:
from 75 weight percent to 85 weight percent water; and
from 15 weight percent to 25 weight percent of the solids component.

3. The composition of claim 1 or claim 2, wherein the solids component comprises:
from 35 weight percent to 55 weight percent of at least one sugar;
from 5.5 weight percent to 9 weight percent of at least one binder;
from 4 weight percent to 7 weight percent of at least one hydroxyalkyl cellulose;
from 25 weight percent to 40 weight percent of at least one water soluble polymer;
from 0.001 weight percent to 3 weight percent of the therapeutic agent medroxyprogesterone acetate;
from 3 weight percent to 20 weight percent of a second polymer that is water soluble or water dispersible; and
from 2 weight percent to 4 weight percent at least one plasticizer.

4. The composition of claim 1 or claim 2, wherein the solids component comprises:
from 35 weight percent to 45 weight percent of at least one sugar;
from 5.5 weight percent to 7.5 weight percent of at least one binder;
from 4 weight percent to 5.5 weight percent of at least one hydroxyalkyl cellulose;
from 20 weight percent to 30 weight percent of at least one water soluble polymer;
from 0.001 weight percent to 3 weight percent of the therapeutic agent medroxyprogesterone acetate;
from 3 weight percent to 20 weight percent of a second polymer that is water soluble or water dispersible; and
from 2.5 weight percent to 3.5 weight percent of at least one plasticizer.

5. The composition of any one of claims 1 to 4, wherein the medroxyprogesterone acetate is in an amount from 0.5 mg to 10 mg.

6. The composition of any one of claims 1 to 5, wherein the polymethacrylate comprises neutral methyacrylic acid esters.

7. The composition of claim 6, wherein the neutral methyacrylic acid esters comprise trimethylammonioethyl methacrylate chloride in a molar ratio of quaternary ammonium groups to neutral ester groups of 1:20.

8. The composition of claim 6, wherein the neutral methyacrylic acid esters comprise trimethylammonioethyl methacrylate chloride in a molar ratio of quaternary ammonium groups to neutral ester groups of 1:40.

9. The composition of claim 6, wherein the neutral methyacrylic acid esters are without any functional groups.

10. The composition of any one of claims 1 to 9, wherein the second polymer is in an amount from 3 weight percent to 20 weight percent of the solids component.

11. The composition of any one of claims 1 to 10 suitable for application by spraying.

12. A solid dosage form comprising a core material and at least one coating disposed thereon, wherein the coating comprises:
from 30 weight percent to 60 weight percent of at least one sugar;
from 5 weight percent to 10 weight percent of at least one binder;
from 3 weight percent to 10 weight percent of at least one hydroxyalkyl cellulose;
from 15 weight percent to 50 weight percent of at least one water soluble polymer;
a therapeutic agent which is medroxyprogesterone acetate in an amount of up to 3 weight percent;
a second polymer that is water soluble or water dispersible, in an amount of up to 20 weight percent; and
optionally, at least one plasticizer in an amount of up to 8 weight percent;
wherein, in the coating:
the sugar comprises sucrose;
the binder comprises microcrystalline cellulose;
the hydroxyalkyl cellulose comprises hydroxypropyl cellulose;
the water soluble polymer comprises hydroxypropyl methylcellulose;
the second polymer comprises a polymethacrylate;
and the plasticizer comprises polyethylene glycol.

13. The solid dosage form of claim 12, wherein the coating comprises:
from 35 weight percent to 55 weight percent of at least one sugar;
from 5.5 weight percent to 9 weight percent of at least one binder;
from 4 weight percent to 7 weight percent of at least one hydroxyalkyl cellulose;
from 25 weight percent to 40 weight percent of at least one water soluble polymer;
from 0.001 weight percent to 3 weight percent of the therapeutic agent medroxyprogesterone acetate;
optionally, from 3 weight percent to 20 weight percent of a second polymer that is water soluble or water dispersible; and
from 2 weight percent to 4 weight percent of at least one plasticizer

14. The solid dosage form of claim 12, wherein the coating comprises:
from 35 weight percent to 45 weight percent of at least one sugar;
from 5.5 weight percent to 7.5 weight percent of at least one binder;
from 4 weight percent to 5.5 weight percent of at least one hydroxyalkyl cellulose;
from 20 weight percent to 30 weight percent of at least one water soluble polymer;
from 0.001 weight percent to 3 weight percent of the therapeutic agent medroxyprogesterone acetate;
optionally, from 3 weight percent to 20 weight percent of a second polymer that is water soluble or water dispersible; and
from 2.5 weight percent to 3.5 weight percent of at least one plasticizer.

15. The solid dosage form of any one of claims 12 to 14, wherein the polymethacrylate comprises neutral methyacrylic acid esters.

16. The solid dosage form of claim 15, wherein the neutral methyacrylic acid esters comprise trimethylammonioethyl methacrylate chloride in a molar ratio of quaternary ammonium groups to neutral ester groups of 1:20.

17. The solid dosage form of claim 15, wherein the neutral methyacrylic acid esters comprise trimethylammonioethyl methacrylate chloride in a molar ratio of quaternary ammonium groups to neutral ester groups of 1:40.

18. The solid dosage form of claim 15, wherein the neutral methyacrylic acid esters are without any functional groups.

19. The solid dosage form of any one of claims 12 to 18, wherein the second polymer of the coating is in an amount from 3 weight percent to 20 weight percent.

20. The solid dosage form of any one of claims 12 to 19, which is in the form of a coated tablet.

21. The solid dosage form of claim 20, further comprising a color coat, a polish coat, or both a color coat and a polish coat.

22. The solid dosage form of claim 20 or claim 21, wherein the coating is disposed directly upon the core material.

23. The solid dosage form of any one of claims 12 to 22, wherein:
the core material comprises from 30 weight percent to 70 weight percent of the dosage form; and
the coating comprises from 30 weight percent to 70 weight percent of the dosage form.

24. The solid dosage form of any one of claims 12 to 22, wherein:
the core material comprises from 40 weight percent to 60 weight percent of the dosage form; and
the coating comprises from 40 weight percent to 60 weight percent of the dosage form.

25. The solid dosage form of claim 23, further comprising:
from 0.5 weight percent to 10 weight percent of a color coat; and
from 0.1 weight percent to 5 weight percent of a polish coat.

26. The solid dosage form of claim 24, further comprising:
from 0.5 weight percent to 10 weight percent of a color coat; and
from 0.1 weight percent to 5 weight percent of a polish coat.

27. The solid dosage form of any one of claims 12 to 20, wherein the core material comprises a therapeutic agent.

28. The solid dosage form of claim 27, wherein the core material further comprises at least one pharmaceutically acceptable excipient.

29. The solid dosage form of claim 28, wherein the excipient of the core material comprises a cellulosic material, a sugar, or a mixture thereof.

30. The solid dosage form of claim 29, wherein the cellulosic material of the excipient of the core material is present in an amount from 15 weight percent to 50 weight percent based on the total weight of the solid dosage form.

31. The solid dosage form of claim 29, wherein the cellulosic material of the excipient of the core material is present in an amount from 18 weight percent to 40 weight percent based on the total weight of the solid dosage form.

32. The solid dosage form of claim 29, wherein the cellulosic material of the excipient of the core material is present in an amount from 40 weight percent to 45 weight percent based on the total weight of the solid dosage form.

33. The solid dosage form of any one of claims 29 to 32, wherein the sugar of the excipient of the core material comprises lactose or lactose monohydrate.

34. The solid dosage form of any one of claims 30 to 33, wherein the pharmaceutically acceptable excipient of the core material comprises a mixture of lactose monohydrate and one or more of hydroxypropyl methylcelluose, microcrystalline cellulose, or sodium carboxymethylcellulose.

35. The solid dosage form of claim 34, wherein the mixture of the excipient of the core material comprises from 4 weight percent to 35 weight percent lactose monohydrate and from 20 weight percent to 40 weight percent cellulosic material based on the total weight of the solid dosage form.

36. The solid dosage form of any one of claims 27 to 35, wherein the therapeutic agent of the core material is a conjugated estrogen or a combination of conjugated estrogens.

37. The solid dosage form of claim 36, wherein the conjugated estrogens are provided as a conjugated estrogens desiccation with lactose.

38. The solid dosage form of claim 37, wherein the conjugated estrogens are present in the conjugated estrogens desiccation with lactose at a concentration of 4.3 weight percent.

39. The solid dosage form of any one of claims 36 to 38, wherein the conjugated estrogens are present in an amount: from 0.1 mg to 5 mg; or from 0.3 mg to 2 mg.

40. The solid dosage form of any one of claims 36 to 39, wherein the conjugated estrogens are present, based on the total weight of the solid dosage form, on a dry weight basis, in an amount: from 0.05 weight percent to 1.0 weight percent; or from 0.1 weight percent to 0.3 weight percent.

41. The solid dosage form of claim 36, wherein based on the total weight of the solid dosage form, on a dry weight basis, the core material comprises:
from 0.1 weight percent to 0.3 weight percent conjugated estrogens;
from 4 weight percent to 35 weight percent lactose monohydrate;
from 5 weight percent to 10 weight percent microcrystalline cellulose;
from 10 weight percent to 35 weight percent hydroxypropyl methyl cellulose; and
from 0 to 1 weight percent of a lubricant.

42. The solid dosage form of claim 41, wherein the coating comprises by weight as a percentage of the solids in the coating:
from 35 weight percent to 55 weight percent sucrose;
from 5.5 weight percent to 9 weight percent microcrystalline cellulose;
from 4 weight percent to 7 weight percent hydroxypropyl cellulose;
from 25 weight percent to 40 weight percent hydroxypropyl methylcellulose;
optionally, from 0.001 weight percent to 3 weight percent of a therapeutic agent;
optionally, from 3 weight percent to 20 weight percent of a second polymer that is water soluble or water dispersible; and
from 2 weight percent to 4 weight percent polyethylene glycol.

43. A solid dosage form of claim 12 comprising:
a tablet core comprising:
11 mg conjugated estrogens desiccation with lactose containing 4.3 weight percent conjugated estrogens;
58 mg lactose monohydrate;
18 mg microcrystalline cellulose;
33 mg hydroxypropyl methylcellulose; and
less than 1 mg of a lubricant;
a coating comprising:
1.5 mg medroxyprogesterone actetate;
37 mg sucrose;
6 mg microcrystalline cellulose;
4.5 mg hydroxypropyl cellulose;
25 mg hydroxypropyl methylcellulose;
3 mg polyethylene glycol 400; and
47 mg polymethacrylate;
a color coat comprising 0.2 mg of a coloring agent; and
a polish coat comprising 2 mg of a polishing agent.

44. A process comprising:
providing a tablet core;
applying to the tablet core a sugar coating composition comprising water and a solids component as defined in any one of claims 1 to 11 to provide a coated tablet core.

45. The process of claim 44, further comprising the step of applying a color coat, a polish coat, or both a color coat and a polish coat to the coated tablet core.

46. The process of claim 44 or claim 45, wherein the tablet core comprises a therapeutic agent and one or more cellulosic materials, wherein the cellulosic materials comprise from 30 weight percent to 50 weight percent of the tablet core.

47. The process of claim 46, wherein the tablet core further comprises from 10 weight percent to 65 weight percent lactose monohydrate, based on the weight of the tablet core.

48. The process of claim 44, wherein the tablet core comprises, based on the weight of the tablet core:
from 5 weight percent to 15 weight percent conjugated estrogens desiccation with lactose containing 4.3 weight percent conjugated estrogens;
from 10 weight percent to 65 weight percent of at least one sugar;
from 10 weight percent to 20 weight percent of at least one binder;
from 15 weight percent to 70 weight percent of at least one water soluble polymer; and
from 0 to 1 weight percent of at least one lubricant.

49. The process of claim 48, wherein in the tablet core: the sugar comprises lactose monohydrate; the binder comprises microcrystalline cellulose; and the water soluble polymer comprises hydroxypropyl methyl cellulose.

50. The process of any one of claims 48 to 49, further comprising the step of applying a color coat, a polish coat, or both a color coat and a polish coat to the coated tablet core.

51. The process of claim 45 or 50, wherein the tablet core is placed in a pan, then sequentially sprayed with the sugar coating composition, a color coat and a polish coat.

52. The process of claim 51, wherein said pan is a perforated pan.

53. The process of claim 52, wherein said perforated pan is side-vented.

54. The process of any one of claims 51 to 53, wherein the sugar coating composition is sprayed at an air flow rate of from 500 cubic feet per minute to 9000 cubic feet per minute.

55. The process of any one of claims 51 to 53, wherein the sugar coating composition is sprayed at an air flow rate of from 1000 cubic feet per minute to 5000 cubic feet per minute.

56. The process of claim 54 or claim 55, wherein the tablet core is at a temperature of from 35 degrees centigrade to 50 degrees centigrade with an inlet air temperature of 50 degrees centigrade to 80 degrees centigrade.

57. The process of claim 44, wherein the tablet core further comprises a conjugated estrogen or a combination of conjugated estrogens.

## Patentansprüche

1. Zusammensetzung in der Form einer wässrigen Suspension, umfassend Wasser und einen Feststoffanteil, welcher:
30 Gewichtsprozent bis 60 Gewichtsprozent von mindestens einem Zucker;
5 Gewichtsprozent bis 10 Gewichtsprozent von mindestens einem Bindemittel;
3 Gewichtsprozent bis 10 Gewichtsprozent von mindestens einer Hydroxyalkylcellulose;
15 Gewichtsprozent bis 50 Gewichtsprozent von mindestens einem wasserlöslichen Polymer;
das therapeutische Mittel Medroxyprogesteronacetat in einer Menge von bis zu 3 Gewichtsprozent;
ein zweites Polymer, welches wasserlöslich oder wasserdispergierbar ist, in einer Menge von bis zu 20 Gewichtsprozent; und
gegebenenfalls mindestens einen Weichmacher in einer Menge von bis zu 8 Gewichtsprozent
umfasst;
wobei
der Zucker Saccharose umfasst;
das Bindemittel mikrokristalline Cellulose umfasst;
die Hydroxyalkylcellulose Hydroxypropylcellulose umfasst;
das wasserlösliche Polymer Hydroxypropylmethylcellulose umfasst;
das zweite Polymer ein Polymethacrylat umfasst;
und der Weichmacher, falls vorhanden, Polyethylenglycol umfasst.

2. Zusammensetzung gemäß Anspruch 1, welche:
75 Gewichtsprozent bis 85 Gewichtsprozent Wasser und
15 Gewichtsprozent bis 25 Gewichtsprozent des Feststoffanteils umfasst.

3. Zusammensetzung gemäß Anspruch 1 oder Anspruch 2, wobei der Feststoffanteil:
35 Gewichtsprozent bis 55 Gewichtsprozent von mindestens einem Zucker;
5,5 Gewichtsprozent bis 9 Gewichtsprozent von mindestens einem Bindemittel;
4 Gewichtsprozent bis 7 Gewichtsprozent von mindestens einer Hydroxyalkylcellulose;
25 Gewichtsprozent bis 40 Gewichtsprozent von mindestens einem wasserlöslichen Polymer;
0,001 Gewichtsprozent bis 3 Gewichtsprozent von dem therapeutischen Mittel Medroxyprogesteronacetat;
3 Gewichtsprozent bis 20 Gewichtsprozent von einem zweiten Polymer, welches wasserlöslich oder wasserdispergierbar ist; und
2 Gewichtsprozent bis 4 Gewichtsprozent von mindestens einem Weichmacher
umfasst.

4. Zusammensetzung gemäß Anspruch 1 oder Anspruch 2, wobei der Feststoffanteil:
35 Gewichtsprozent bis 45 Gewichtsprozent von mindestens einem Zucker;
5,5 Gewichtsprozent bis 7,5 Gewichtsprozent von mindestens einem Bindemittel;
4 Gewichtsprozent bis 5,5 Gewichtsprozent von mindestens einer Hydroxyalkylcellulose;
20 Gewichtsprozent bis 30 Gewichtsprozent von mindestens einem wasserlöslichen Polymer;
0,001 Gewichtsprozent bis 3 Gewichtsprozent von dem therapeutischen Mittel Medroxyprogesteronacetat;
3 Gewichtsprozent bis 20 Gewichtsprozent von einem zweiten Polymer, welches wasserlöslich oder wasserdispergierbar ist; und
2,5 Gewichtsprozent bis 3,5 Gewichtsprozent von mindestens einem Weichmacher umfasst.

5. Zusammensetzung gemäß einem der Ansprüche 1 bis 4, wobei das Medroxyprogesteronacetat in einer Menge von 0,5 mg bis 10 mg vorliegt.

6. Zusammensetzung gemäß einem der Ansprüche 1 bis 5, wobei das Polymethacrylat neutrale Methacrylsäureester umfasst.

7. Zusammensetzung gemäß Anspruch 6, wobei die neutralen Methacrylsäureester Trimethylammoniumethylmethacrylatchlorid in einem Molverhältnis von quaternären Ammoniumgruppen zu neutralen Estergruppen von 1:20 umfassen.

8. Zusammensetzung gemäß Anspruch 6, wobei die neutralen Methacrylsäureester Trimethylammoniumethylmethacrylatchlorid in einem Molverhältnis von quaternären Ammoniumgruppen zu neutralen Estergruppen von 1:40 umfassen.

9. Zusammensetzung gemäß Anspruch 6, wobei die neutralen Methacrylsäureester ohne jegliche funktionellen Gruppen sind.

10. Zusammensetzung gemäß einem der Ansprüche 1 bis 9, wobei das zweite Polymer in einer Menge von 3 Gewichtsprozent bis 20 Gewichtsprozent des Feststoffanteils vorliegt.

11. Zusammensetzung gemäß einem der Ansprüche 1 bis 10, welche zur Anwendung durch Sprühen geeignet ist.

12. Feste Dosierungsform, umfassend ein Kernmaterial und mindestens eine darauf angeordnete Beschichtung, wobei die Beschichtung:
30 Gewichtsprozent bis 60 Gewichtsprozent von mindestens einem Zucker;
5 Gewichtsprozent bis 10 Gewichtsprozent von mindestens einem Bindemittel;
3 Gewichtsprozent bis 10 Gewichtsprozent von mindestens einer Hydroxyalkylcellulose;
15 Gewichtsprozent bis 50 Gewichtsprozent von mindestens einem wasserlöslichen Polymer;
ein therapeutisches Mittel, welches Medroxyprogesteronacetat ist, in einer Menge von bis zu 3 Gewichtsprozent;
ein zweites Polymer, welches wasserlösliche oder wasserdispergierbar ist, in einer Menge von bis zu 20 Gewichtsprozent; und
gegebenenfalls mindestens einen Weichmacher in einer Menge von bis zu 8 Gewichtsprozent;
umfasst,
wobei in der Beschichtung:
der Zucker Saccharose umfasst;
das Bindemittel mikrokristalline Cellulose umfasst;
die Hydroxyalkylcellulose Hydroxypropylcellulose umfasst;
das wasserlösliche Polymer Hydroxypropylmethylcellulose umfasst;
das zweite Polymer ein Polymethacrylat umfasst;
und der Weichmacher Polyethylenglycol
umfasst.

13. Feste Dosierungsform gemäß Anspruch 12, wobei die Beschichtung:
35 Gewichtsprozent bis 55 Gewichtsprozent von mindestens einem Zucker;
5,5 Gewichtsprozent bis 9 Gewichtsprozent von mindestens einem Bindemittel;
4 Gewichtsprozent bis 7 Gewichtsprozent von mindestens einer Hydroxyalkylcellulose;
25 Gewichtsprozent bis 40 Gewichtsprozent von mindestens einem wasserlöslichen Polymer;
0,001 Gewichtsprozent bis 3 Gewichtsprozent von dem therapeutischen Mittel Medroxyprogesteronacetat;
gegebenenfalls 3 Gewichtsprozent bis 20 Gewichtsprozent von einem zweiten Polymer, welches wasserlöslich oder wasserdispergierbar ist; und
2 Gewichtsprozent bis 4 Gewichtsprozent von mindestens einem Weichmacher
umfasst.

14. Feste Dosierungsform gemäß Anspruch 12, wobei die Beschichtung:
35 Gewichtsprozent bis 45 Gewichtsprozent von mindestens einem Zucker;
5,5 Gewichtsprozent bis 7,5 Gewichtsprozent von mindestens einem Bindemittel;
4 Gewichtsprozent bis 5,5 Gewichtsprozent von mindestens einer Hydroxyalkylcellulose;
20 Gewichtsprozent bis 30 Gewichtsprozent von mindestens einem wasserlöslichen Polymer;
0,001 Gewichtsprozent bis 3 Gewichtsprozent von dem therapeutischen Mittel Medroxyprogesteronacetat;
gegebenenfalls 3 Gewichtsprozent bis 20 Gewichtsprozent von einem zweiten Polymer, welches wasserlöslich oder wasserdispergierbar ist;
2,5 Gewichtsprozent bis 3,5 Gewichtsprozent von mindestens einem Weichmacher
umfasst.

15. Feste Dosierungsform gemäß einem der Ansprüche 12 bis 14, wobei das Polymethacrylat neutrale Methacrylsäureester umfasst.

16. Feste Dosierungsform gemäß Anspruch 15, wobei die neutralen Methacrylsäureester Trimethylammoniumethylmethacrylatchlorid in einem Molverhältnis von quaternären Ammoniumgruppen zu neutralen Estergruppen von 1:20 umfassen.

17. Feste Dosierungsform gemäß Anspruch 15, wobei die neutralen Methacrylsäureester Trimethylammoniumethylmethacrylatchlorid in einem Molverhältnis von quaternären Ammoniumgruppen zu neutralen Estergruppen von 1:40 umfassen.

18. Feste Dosierungsform gemäß Anspruch 15, wobei die neutralen Methacrylsäureester ohne jegliche funktionellen Gruppen sind.

19. Feste Dosierungsform gemäß einem der Ansprüche 12 bis 18, wobei das zweite Polymer der Beschichtung in einer Menge von 3 Gewichtsprozent bis 20 Gewichtsprozent vorliegt.

20. Feste Dosierungsform gemäß einem der Ansprüche 12 bis 19, welche in der Form einer beschichteten Tablette vorliegt.

21. Feste Dosierungsform gemäß Anspruch 20, zusätzlich umfassend einen Farbüberzug, einen Hochglanzüberzug oder sowohl einen Farbüberzug als auch einen Hochglanzüberzug.

22. Feste Dosierungsform gemäß Anspruch 20 oder Anspruch 21, wobei die Beschichtung direkt auf dem Kernmaterial angeordnet ist.

23. Feste Dosierungsform gemäß einem der Ansprüche 12 bis 22, wobei:
das Kernmaterial 30 Gewichtsprozent bis 70 Gewichtsprozent der Dosierungsform umfasst; und
die Beschichtung 30 Gewichtsprozent bis 70 Gewichtsprozent der Dosierungsform umfasst.

24. Feste Dosierungsform gemäß einem der Ansprüche 12 bis 22, wobei:
das Kernmaterial 40 Gewichtsprozent bis 60 Gewichtsprozent von der Dosierungsform umfasst; und
die Beschichtung 40 Gewichtsprozent bis 60 Gewichtsprozent von der Dosierungsform umfasst.

25. Feste Dosierungsform gemäß Anspruch 23, zusätzlich umfassend:
0,5 Gewichtsprozent bis 10 Gewichtsprozent von einem Farbüberzug; und
0,1 Gewichtsprozent bis 5 Gewichtsprozent von einem Hochglanzüberzug.

26. Feste Dosierungsform gemäß Anspruch 24, zusätzlich umfassend:
0,5 Gewichtsprozent bis 10 Gewichtsprozent von einem Farbüberzug; und
0,1 Gewichtsprozent bis 5 Gewichtsprozent von einem Hochglanzüberzug.

27. Feste Dosierungsform gemäß einem der Ansprüche 12 bis 20, wobei das Kernmaterial ein therapeutisches Mittel umfasst.

28. Feste Dosierungsform gemäß Anspruch 27, wobei das Kernmaterial zusätzlich mindestens einen pharmazeutisch annehmbaren Exzipienten umfasst.

29. Feste Dosierungsform gemäß Anspruch 28, wobei der Exzipient des Kernmaterials ein Cellulosematerial, ein Zucker oder eine Mischung davon umfasst.

30. Feste Dosierungsform gemäß Anspruch 29, wobei das Cellulosematerial von dem Exzipienten des Kernmaterials in einer Menge von 15 Gewichtsprozent bis 50 Gewichtsprozent, basierend auf dem Gesamtgewicht der festen Dosierungsform, vorliegt.

31. Feste Dosierungsform gemäß Anspruch 29, wobei das Cellulosematerial von dem Exzipienten des Kernmaterials in einer Menge von 18 Gewichtsprozent bis 40 Gewichtsprozent, basierend auf dem Gesamtgewicht der festen Dosierungsform, vorliegt.

32. Feste Dosierungsform gemäß Anspruch 29, wobei das Cellulosematerial von dem Exzipienten des Kernmaterials in einer Menge von 40 Gewichtsprozent bis 45 Gewichtsprozent, basierend auf dem Gesamtgewicht der festen Dosierungsform, vorliegt.

33. Fest Dosierungsform gemäß einem der Ansprüche 29 bis 32, wobei der Zucker von dem Exzipienten des Kernmaterials Lactose oder Lactosemonohydrat umfasst.

34. Feste Dosierungsform gemäß einem der Ansprüche 30 bis 33, wobei der pharmazeutisch annehmbare Exzipient des Kernmaterials eine Mischung aus Lactosemonohydrat und einer oder mehrerer aus Hydroxypropylmethylcellulose, mikrokristalline Cellulose oder Natriumcarboxymethylcellulose umfasst.

35. Feste Dosierungsform gemäß Anspruch 34, wobei die Mischung von dem Exzipienten des Kernmaterials 4 Gewichtsprozent bis 35 Gewichtsprozent Lactosemonoydrat und 20 Gewichtsprozent bis 40 Gewichtsprozent Cellulosematerial, basierend auf dem Gesamtgewicht der festen Dosierungsform, umfasst.

36. Feste Dosierungsform gemäß einem der Ansprüche 27 bis 35, wobei das therapeutische Mittel des Kernmaterials ein konjugiertes Östrogen oder eine Kombination aus konjugierten Östrogenen ist.

37. Feste Dosierungsform gemäß Anspruch 36, wobei die konjugierten Östrogene als eine Konjugierte-Östrogene-Trocknung mit Lactose bereitgestellt sind.

38. Feste Dosierungsform gemäß Anspruch 37, wobei die konjugierten Östrogene in der Konjugierte-Östrogene-Trocknung mit Lactose mit einer Konzentration von 4,3 Gewichtsprozent vorliegen.

39. Feste Dosierungsform gemäß einem der Ansprüche 36 bis 38, wobei die konjugierten Östrogene in einer Menge: von 0,1 mg bis 5 mg oder von 0,3 mg bis 2 mg vorliegen.

40. Feste Dosierungsform gemäß einem der Ansprüche 36 bis 39, wobei die konjugierten Östrogene, basierend auf dem Gesamtgewicht der festen Dosierungsform, auf einer Trockengewichtsbasis in einer Menge: von 0,05 Gewichtsprozent bis 1,0 Gewichtsprozent oder von 0,1 Gewichtsprozent bis 0,3 Gewichtsprozent vorliegen.

41. Feste Dosierungsform gemäß Anspruch 36, wobei basierend auf dem Gesamtgewicht der festen Dosierungsform auf einer Trockengewichtsbasis das Kernmaterial:
0,1 Gewichtsprozent bis 0,3 Gewichtsprozent konjugierte Östrogene;
4 Gewichtsprozent bis 35 Gewichtsprozent Lactosemonohydrat;
5 Gewichtsprozent bis 10 Gewichtsprozent mikrokristalline Cellulose;
10 Gewichtsprozent bis 35 Gewichtsprozent Hydroxypropylmethylcellulose; und
0 bis 1 Gewichtsprozent eines Schmiermittels
umfasst.

42. Feste Dosierungsform gemäß Anspruch 41, wobei die Beschichtung als Gewichtsprozent des Feststoffs in der Beschichtung:
35 Gewichtsprozent bis 55 Gewichtsprozent Saccharose;
5,5 Gewichtsprozent bis 9 Gewichtsprozent mikrokristalline Cellulose;
4 Gewichtsprozent bis 7 Gewichtsprozent Hydroxypropylcellulose;
25 Gewichtsprozent bis 40 Gewichtsprozent Hydroxypropylmethylcellulose;
gegebenenfalls 0,001 Gewichtsprozent bis 3 Gewichtsprozent von einem therapeutischen Mittel;
gegebenenfalls 3 Gewichtsprozent bis 20 Gewichtsprozent von einem zweiten Polymer, welches wasserlöslich oder wasserdispergierbar ist; und
2 Gewichtsprozent bis 4 Gewichtsprozent Polyethylenglycol umfasst.

43. Feste Dosierungsform gemäß Anspruch 12, umfassend:
einen Tablettenkern, umfassend:
11 mg Konjugierte-Östrogene-Trocknung mit Lactose, welche 4,3 Gewichtsprozent konjugierte Östrogene enthält;
58 mg Lactosemonohydrat;
18 mg mikrokristalline Cellulose;
33 mg Hydroxypropylmethylcellulose; und
weniger als 1 mg von einem Schmiermittel;
eine Beschichtung, umfassend:
1,5 mg Medroxyprogesteronacetat;
37 mg Saccharose;
6 mg mikrokristalline Cellulose;
4,5 mg Hydroxypropylcellulose;
25 mg Hydroxypropylmethylcellulose;
3 mg Polyethylenglycol 400; und
47 mg Polymethacrylat;
einen Farbüberzug, umfassend 0,2 mg von einem Färbemittel; und
einen Hochglanzüberzug, umfassend 2 mg von einem Hochglanzmittel.

44. Verfahren, umfassend:
Bereitstellen eines Tablettenkerns;
Auftragen auf den Tablettenkern eine Zuckerbeschichtungszusammensetzung, umfassend Wasser und einen Feststoffanteil, wie in einem der Ansprüche 1 bis 11 definiert, um einen beschichteten Tablettenkern bereitzustellen.

45. Verfahren gemäß Anspruch 44, zusätzlich umfassend den Schritt des Auftragens eines Farbüberzugs, eines Hochglanzüberzugs oder sowohl eines Farbüberzugs als auch eines Hochglanzüberzugs auf den beschichteten Tablettenkern.

46. Verfahren gemäß Anspruch 44 oder 45, wobei der Tablettenkern ein therapeutisches Mittel und ein oder mehrere Cellulosematerial(ien) umfasst, wobei die Cellulosematerialien 30 Gewichtsprozent bis 50 Gewichtsprozent von dem Tablettenkern umfassen.

47. Verfahren gemäß Anspruch 46, wobei der Tablettenkern zusätzlich 10 Gewichtsprozent bis 65 Gewichtsprozent Lactosemonohydrat, basierend auf dem Gewicht des Tablettenkerns, umfasst.

48. Verfahren gemäß Anspruch 44, wobei der Tablettenkern, basierend auf dem Gewicht des Tablettenkerns:
5 Gewichtsprozent bis 15 Gewichtsprozent Konjugierte-Östrogene-Trocknung mit Lactose, welche 4,3 Gewichtsprozent konjugierte Östrogene enthält;
10 Gewichtsprozent bis 65 Gewichtsprozent von mindestens einem Zucker;
10 Gewichtsprozent bis 20 Gewichtsprozent von mindestens einem Bindemittel;
15 Gewichtsprozent bis 70 Gewichtsprozent von mindestens einem wasserlöslichen Polymer; und
0 bis 1 Gewichtsprozent von mindestens einem Schmiermittel umfasst.

49. Verfahren gemäß Anspruch 48, wobei in dem Tablettenkern: der Zucker Lactosemonohydrat umfasst; das Bindemittel mikrokristalline Cellulose umfasst und das wasserlösliche Polymer Hydroxypropylmethylcellulose umfasst.

50. Verfahren gemäß einem der Ansprüche 48 bis 49, zusätzlich umfassend den Schritt des Auftragens eines Farbüberzugs, eines Hochglanzüberzugs oder sowohl eines Farbüberzugs als auch eines Hochglanzüberzugs auf den beschichteten Tablettenkern.

51. Verfahren gemäß Anspruch 45 oder 50, wobei der Tablettenkern in einer Pfanne platziert wird, dann aufeinanderfolgend mit der Zuckerbeschichtungszusammensetzung, dem Farbüberzug und dem Hochglanzüberzug besprüht wird.

52. Verfahren gemäß Anspruch 51, wobei genannte Pfanne eine perforierte Pfanne ist.

53. Verfahren gemäß Anspruch 52, wobei genannte perforierte Pfanne seitlich belüftet ist.

54. Verfahren gemäß einem der Ansprüche 51 bis 53, wobei die Zuckerbeschichtungszusammensetzung mit einem Luftdurchsatz von 500 Kubikfuß pro Minute bis 9.000 Kubikfuß pro Minute gesprüht wird.

55. Verfahren gemäß einem der Ansprüche 51 bis 53, wobei die Zuckerbeschichtungszusammensetzung mit einem Luftdurchsatz von 1.000 Kubikfuß pro Minute bis 5.000 Kubikfuß pro Minute gesprüht wird.

56. Verfahren gemäß Anspruch 54 oder Anspruch 55, wobei der Tablettenkern bei einer Temperatur von 35 Grad Celsius bis 50 Grad Celsius mit einer Lufteinlasstemperatur von 50 Grad Celsius bis 80 Grad Celsius ist.

57. Verfahren gemäß Anspruch 44, wobei der Tablettenkern zusätzlich ein konjugiertes Östrogen oder eine Kombination aus konjugierten Östrogenen umfasst.

## Revendications

1. Composition sous la forme d'une suspension aqueuse comprenant de l'eau et un composant solide qui comprend :
de 30 pour cent en poids à 60 pour cent en poids d'au moins un sucre ;
de 5 pour cent en poids à 10 pour cent en poids d'au moins un liant ;
de 3 pour cent en poids à 10 pour cent en poids d'au moins une hydroxyalkylcellulose ;
de 15 pour cent en poids à 50 pour cent en poids d'au moins un polymère hydrosoluble ;
l'agent thérapeutique acétate de médroxy-progestérone en une quantité allant jusqu'à 3 pour cent en poids ;
un second polymère qui est soluble dans l'eau ou dispersible dans l'eau, en une quantité allant jusqu'à 20 pour cent en poids ; et
optionnellement, au moins un plastifiant en une quantité allant jusqu'à 8 pour cent en poids ;
dans laquelle
le sucre comprend du saccharose ;
le liant comprend de la cellulose microcristalline ;
l'hydroxyalkylcellulose comprend de l'hydroxypropylcellulose ;
le polymère hydrosoluble comprend de l'hydroxypropylméthylcellulose ;
le second polymère comprend un polyméthacrylate ;
et le plastifiant, quand il est présent, comprend un polyéthylène glycol.

2. Composition selon la revendication 1, qui comprend :
de 75 pour cent en poids à 85 pour cent en poids d'eau ; et
de 15 pour cent en poids à 25 pour cent en poids du composant solide.

3. Composition selon la revendication 1 ou la revendication 2, dans laquelle le composant solide comprend :
de 35 pour cent en poids à 55 pour cent en poids d'au moins un sucre ;
de 5,5 pour cent en poids à 9 pour cent en poids d'au moins un liant ;
de 4 pour cent en poids à 7 pour cent en poids d'au moins une hydroxyalkylcellulose ;
de 25 pour cent en poids à 40 pour cent en poids d'au moins un polymère hydrosoluble ;
de 0,001 pour cent en poids à 3 pour cent en poids de l'agent thérapeutique acétate de médroxy-progestérone ;
de 3 pour cent en poids à 20 pour cent en poids d'un second polymère qui est soluble dans l'eau ou dispersible dans l'eau ; et
de 2 pour cent en poids à 4 pour cent en poids d'au moins un plastifiant.

4. Composition selon la revendication 1 ou la revendication 2, dans laquelle le composant solide comprend :
de 35 pour cent en poids à 45 pour cent en poids d'au moins un sucre ;
de 5,5 pour cent en poids à 7,5 pour cent en poids d'au moins un liant ;
de 4 pour cent en poids à 5,5 pour cent en poids d'au moins une hydroxyalkylcellulose ;
de 20 pour cent en poids à 30 pour cent en poids d'au moins un polymère hydrosoluble ;
de 0,001 pour cent en poids à 3 pour cent en poids de l'agent thérapeutique acétate de médroxy-progestérone ;
de 3 pour cent en poids à 20 pour cent en poids d'un second polymère qui est soluble dans l'eau ou dispersible dans l'eau ; et
de 2,5 pour cent en poids à 3,5 pour cent en poids d'au moins un plastifiant.

5. Composition selon l'une quelconque des revendications 1 à 4, dans laquelle l'acétate de médroxyprogestérone est en une quantité de 0,5 mg à 10 mg.

6. Composition selon l'une quelconque des revendications 1 à 5, dans laquelle le polyméthacrylate comprend des esters neutres d'acide méthyacrylique.

7. Composition selon la revendication 6, dans laquelle les esters neutres d'acide méthyacrylique comprennent le chlorure de méthacrylate de triméthylammonioéthyle à un rapport molaire groupes ammonium quaternaire sur groupes ester neutre de 1/20.

8. Composition selon la revendication 6, dans laquelle les esters neutres d'acide méthyacrylique comprennent le chlorure de méthacrylate de triméthylammonioéthyle à un rapport molaire groupes ammonium quaternaire sur groupes ester neutre de 1/40.

9. Composition selon la revendication 6, dans laquelle les esters neutres d'acide méthyacrylique sont sans aucun groupe fonctionnel.

10. Composition selon l'une quelconque des revendications 1 à 9, dans laquelle le second polymère est en une quantité de 3 pour cent en poids à 20 pour cent en poids du composant solide.

11. Composition selon l'une quelconque des revendications 1 à 10, appropriée pour une application par pulvérisation.

12. Forme galénique solide comprenant un matériau de noyau et au moins un enrobage disposé dessus, dans laquelle l'enrobage comprend :
de 30 pour cent en poids à 60 pour cent en poids d'au moins un sucre ;
de 5 pour cent en poids à 10 pour cent en poids d'au moins un liant ;
de 3 pour cent en poids à 10 pour cent en poids d'au moins une hydroxyalkylcellulose ;
de 15 pour cent en poids à 50 pour cent en poids d'au moins un polymère hydrosoluble ;
un agent thérapeutique qui est l'acétate de médroxyprogestérone en une quantité allant jusqu'à 3 pour cent en poids ;
un second polymère qui est soluble dans l'eau ou dispersible dans l'eau, en une quantité allant jusqu'à 20 pour cent en poids ; et
optionnellement, au moins un plastifiant en une quantité allant jusqu'à 8 pour cent en poids ;
dans laquelle, dans l'enrobage :
le sucre comprend du saccharose ;
le liant comprend de la cellulose microcristalline ;
l'hydroxyalkylcellulose comprend de l'hydroxypropylcellulose ;
le polymère hydrosoluble comprend de l'hydroxypropylméthylcellulose ;
le second polymère comprend un polyméthacrylate ;
et le plastifiant comprend un polyéthylène glycol.

13. Forme galénique solide selon la revendication 12, dans laquelle l'enrobage comprend :
de 35 pour cent en poids à 55 pour cent en poids d'au moins un sucre ;
de 5,5 pour cent en poids à 9 pour cent en poids d'au moins un liant ;
de 4 pour cent en poids à 7 pour cent en poids d'au moins une hydroxyalkylcellulose ;
de 25 pour cent en poids à 40 pour cent en poids d'au moins un polymère hydrosoluble ;
de 0,001 pour cent en poids à 3 pour cent en poids de l'agent thérapeutique acétate de médroxy-progestérone ;
optionnellement, de 3 pour cent en poids à 20 pour cent en poids d'un second polymère qui est soluble dans l'eau ou dispersible dans l'eau ; et
de 2 pour cent en poids à 4 pour cent en poids d'au moins un plastifiant.

14. Forme galénique solide selon la revendication 12, dans laquelle l'enrobage comprend :
de 35 pour cent en poids à 45 pour cent en poids d'au moins un sucre ;
de 5,5 pour cent en poids à 7,5 pour cent en poids d'au moins un liant ;
de 4 pour cent en poids à 5,5 pour cent en poids d'au moins une hydroxyalkylcellulose ;
de 20 pour cent en poids à 30 pour cent en poids d'au moins un polymère hydrosoluble ;
de 0,001 pour cent en poids à 3 pour cent en poids de l'agent thérapeutique acétate de médroxy-progestérone ;
optionnellement, de 3 pour cent en poids à 20 pour cent en poids d'un second polymère qui est soluble dans l'eau ou dispersible dans l'eau ; et
de 2,5 pour cent en poids à 3,5 pour cent en poids d'au moins un plastifiant.

15. Forme galénique solide selon l'une quelconque des revendications 12 à 14, dans laquelle le polyméthacrylate comprend des esters neutres d'acide méthyacrylique.

16. Forme galénique solide selon la revendication 15, dans laquelle les esters neutres d'acide méthyacrylique comprennent le chlorure de méthacrylate de triméthylammonioéthyle à un rapport molaire groupes ammonium quaternaire sur groupes ester neutre de 1/20.

17. Forme galénique solide selon la revendication 15, dans laquelle les esters neutres d'acide méthyacrylique comprennent le chlorure de méthacrylate de triméthylammonioéthyle à un rapport molaire groupes ammonium quaternaire sur groupes ester neutre de 1/40.

18. Forme galénique solide selon la revendication 15, dans laquelle les esters neutres d'acide méthyacrylique sont sans aucun groupe fonctionnel.

19. Forme galénique solide selon l'une quelconque des revendications 12 à 18, dans laquelle le second polymère de l'enrobage est en une quantité de 3 pour cent en poids à 20 pour cent en poids.

20. Forme galénique solide selon l'une quelconque des revendications 12 à 19, qui est sous la forme d'un comprimé enrobé.

21. Forme galénique solide selon la revendication 20, comprenant en outre une couche de couleur, une couche de vernis, ou à la fois une couche de couleur et une couche de vernis.

22. Forme galénique solide selon la revendication 20 ou la revendication 21, dans laquelle l'enrobage est disposé directement sur le matériau de noyau.

23. Forme galénique solide selon l'une quelconque des revendications 12 à 22, dans laquelle :
le matériau de noyau comprend de 30 pour cent en poids à 70 pour cent en poids de la forme galénique ; et
l'enrobage comprend de 30 pour cent en poids à 70 pour cent en poids de la forme galénique.

24. Forme galénique solide selon l'une quelconque des revendications 12 à 22, dans laquelle :
le matériau de noyau comprend de 40 pour cent en poids à 60 pour cent en poids de la forme galénique ; et
l'enrobage comprend de 40 pour cent en poids à 60 pour cent en poids de la forme galénique.

25. Forme galénique solide selon la revendication 23, comprenant en outre :
de 0,5 pour cent en poids à 10 pour cent en poids d'une couche de couleur ; et
de 0,1 pour cent en poids à 5 pour cent en poids d'une couche de vernis.

26. Forme galénique solide selon la revendication 24, comprenant en outre :
de 0,5 pour cent en poids à 10 pour cent en poids d'une couche de couleur ; et
de 0,1 pour cent en poids à 5 pour cent en poids d'une couche de vernis.

27. Forme galénique solide selon l'une quelconque des revendications 12 à 20, dans laquelle le matériau de noyau comprend un agent thérapeutique.

28. Forme galénique solide selon la revendication 27, dans laquelle le matériau de noyau comprend au moins un excipient pharmaceutiquement acceptable.

29. Forme galénique solide selon la revendication 28, dans laquelle l'excipient du matériau de noyau comprend un matériau cellulosique, un sucre, ou un mélange de ceux-ci.

30. Forme galénique solide selon la revendication 29, dans laquelle le matériau cellulosique de l'excipient du matériau de noyau est présent en une quantité de 15 pour cent en poids à 50 pour cent en poids sur la base du poids total de la forme galénique solide.

31. Forme galénique solide selon la revendication 29, dans laquelle le matériau cellulosique de l'excipient du matériau de noyau est présent en une quantité de 18 pour cent en poids à 40 pour cent en poids sur la base du poids total de la forme galénique solide.

32. Forme galénique solide selon la revendication 29, dans laquelle le matériau cellulosique de l'excipient du matériau de noyau est présent en une quantité de 40 pour cent en poids à 45 pour cent en poids sur la base du poids total de la forme galénique solide.

33. Forme galénique solide selon l'une quelconque des revendications 29 à 32, dans laquelle le sucre de l'excipient du matériau de noyau comprend du lactose ou du lactose monohydraté.

34. Forme galénique solide selon l'une quelconque des revendications 30 à 33, dans laquelle l'excipient pharmaceutiquement acceptable du matériau de noyau comprend un mélange de lactose monohydraté et un ou plusieurs parmi l'hydroxypropylméthylcellulose, la cellulose microcristalline ou la carboxyméthyl-cellulose sodique.

35. Forme galénique solide selon la revendication 34, dans laquelle le mélange de l'excipient du matériau de noyau comprend 4 pour cent en poids à 35 pour cent en poids de lactose monohydraté et 20 pour cent en poids à 40 pour cent en poids de matériau cellulosique sur la base du poids total de la forme galénique solide.

36. Forme galénique solide selon l'une quelconque des revendications 27 à 35, dans laquelle l'agent thérapeutique du matériau de noyau est un oestrogène conjugué ou une combinaison d'oestrogènes conjugués.

37. Forme galénique solide selon la revendication 36, dans laquelle les oestrogènes conjugués sont fournis sous la forme d'une dessiccation d'oestrogènes conjugués avec du lactose.

38. Forme galénique solide selon la revendication 37, dans laquelle les oestrogènes conjugués sont présents dans la dessiccation d'oestrogènes conjugués avec du lactose à une concentration de 4,3 pour cent en poids.

39. Forme galénique solide selon l'une quelconque des revendications 36 à 38, dans laquelle les oestrogènes conjugués sont présents en une quantité : de 0,1 mg à 5 mg ; ou de 0,3 mg à 2 mg.

40. Forme galénique solide selon l'une quelconque des revendications 36 à 39, dans laquelle les oestrogènes conjugués sont présents, sur la base du poids total de la forme galénique solide, sur une base en poids sec, en une quantité : de 0,05 pour cent en poids à 1,0 pour cent en poids ; ou de 0,1 pour cent en poids à 0,3 pour cent en poids.

41. Forme galénique solide selon la revendication 36, dans laquelle, sur la base du poids total de la forme galénique solide, sur une base en poids sec, le matériau de noyau comprend :
de 0,1 pour cent en poids à 0,3 pour cent en poids d'oestrogènes conjugués ;
de 4 pour cent en poids à 35 pour cent en poids de lactose monohydraté ;
de 5 pour cent en poids à 10 pour cent en poids de cellulose microcristalline ;
de 10 pour cent en poids à 35 pour cent en poids d'hydroxypropylméthylcellulose ; et
de 0 à 1 pour cent en poids d'un lubrifiant.

42. Forme galénique solide selon la revendication 41, dans laquelle l'enrobage comprend en poids sous la forme d'un pourcentage des solides dans l'enrobage :
de 35 pour cent en poids à 55 pour cent en poids de saccharose ;
de 5,5 pour cent en poids à 9 pour cent en poids de cellulose microcristalline ;
de 4 pour cent en poids à 7 pour cent en poids d'hydroxypropylcellulose ;
de 25 pour cent en poids à 40 pour cent en poids d'hydroxypropylméthylcellulose ;
optionnellement, de 0,001 pour cent en poids à 3 pour cent en poids d'un agent thérapeutique ;
optionnellement, de 3 pour cent en poids à 20 pour cent en poids d'un second polymère qui est soluble dans l'eau ou dispersible dans l'eau ; et
de 2 pour cent en poids à 4 pour cent en poids d'un polyéthylène glycol.

43. Forme galénique solide selon la revendication 12, comprenant :
un noyau de comprimé comprenant :
11 mg d'une dessiccation d'oestrogènes conjugués avec du lactose contenant 4,3 pour cent en poids d'oestrogènes conjugués ;
58 mg de lactose monohydraté ;
18 mg de cellulose microcristalline ;
33 mg d'hydroxypropylméthylcellulose ; et
moins de 1 mg d'un lubrifiant ;
un enrobage comprenant :
1,5 mg d'acétate de médroxyprogestérone ;
37 mg de saccharose ;
6 mg de cellulose microcristalline ;
4,5 mg d'hydroxypropylcellulose ;
25 mg d'hydroxypropylméthylcellulose ;
3 mg de polyéthylène glycol 400 ; et
47 mg d'un polyméthacrylate ;
une couche de couleur comprenant 0,2 mg d'un agent colorant ; et
une couche de vernis comprenant 2 mg d'un agent de lustrage.

44. Procédé comprenant :
la fourniture d'un noyau de comprimé ;
l'application sur le noyau de comprimé d'une composition d'enrobage à base de sucre comprenant de l'eau et un composant solide tel que défini dans l'une quelconque des revendications 1 à 11 pour fournir un noyau de comprimé enrobé.

45. Procédé selon la revendication 44, comprenant en outre l'étape d'application d'une couche de couleur, d'une couche de vernis, ou à la fois d'une couche de couleur et d'une couche de vernis sur le noyau de comprimé enrobé.

46. Procédé selon la revendication 44 ou la revendication 45, dans lequel le noyau de comprimé comprend un agent thérapeutique et un ou plusieurs matériaux cellulosiques, dans lequel les matériaux cellulosiques comprennent de 30 pour cent en poids à 50 pour cent en poids du noyau de comprimé.

47. Procédé selon la revendication 46, dans lequel le noyau de comprimé comprend en outre de 10 pour cent en poids à 65 pour cent en poids de lactose monohydraté, sur la base du poids du noyau de comprimé.

48. Procédé selon la revendication 44, dans lequel le noyau de comprimé comprend, sur la base du poids du noyau de comprimé :
de 5 pour cent en poids à 15 pour cent en poids d'une dessiccation d'oestrogènes conjugués avec du lactose contenant 4,3 pour cent en poids d'oestrogènes conjugués ;
de 10 pour cent en poids à 65 pour cent en poids d'au moins un sucre ;
de 10 pour cent en poids à 20 pour cent en poids d'au moins un liant ;
de 15 pour cent en poids à 70 pour cent en poids d'au moins un polymère hydrosoluble ; et
de 0 à 1 pour cent en poids d'au moins un lubrifiant.

49. Procédé selon la revendication 48, dans lequel, dans le noyau de comprimé : le sucre comprend du lactose monohydraté ; le liant comprend de la cellulose microcristalline ; et le polymère hydrosoluble comprend de l'hydroxypropylméthylcellulose.

50. Procédé selon l'une quelconque des revendications 48 à 49, comprenant en outre l'étape d'application d'une couche de couleur, d'une couche de vernis, ou à la fois d'une couche de couleur et d'une couche de vernis sur le noyau de comprimé enrobé.

51. Procédé selon la revendication 45 ou 50, dans lequel le noyau de comprimé est placé dans une turbine, puis pulvérisé de manière séquentielle avec la composition d'enrobage à base de sucre, une couche de couleur et une couche de vernis.

52. Procédé selon la revendication 51, dans lequel ladite turbine est une turbine à paroi perforée.

53. Procédé selon la revendication 52, dans lequel ladite turbine à paroi perforée est ventilée latéralement.

54. Procédé selon l'une quelconque des revendications 51 à 53, dans lequel la composition d'enrobage à base de sucre est pulvérisée à un débit d'air de 500 pieds cubes par minute à 9000 pieds cubes par minute.

55. Procédé selon l'une quelconque des revendications 51 à 53, dans lequel la composition d'enrobage à base de sucre est pulvérisée à un débit d'air de 1000 pieds cubes par minute à 5000 pieds cubes par minute.

56. Procédé selon la revendication 54 ou la revendication 55, dans lequel le noyau de comprimé est à une température de 35 degrés centigrades à 50 degrés centigrades avec une température d'entrée d'air de 50 degrés centigrades à 80 degrés centigrades.

57. Procédé selon la revendication 44, dans lequel le noyau de comprimé comprend en outre un estrogène conjugué ou une combinaison d'oestrogènes conjugués.
